# EUROPEAN PATENT SPECIFICATION

(11) **EP 2 402 038 B1**
(45) Date of publication and mention of the grant of the patent: **25.05.2016**
(21) Application number: 10168341.5
(22) Date of filing: 02.07.2010
(51) Int. Cl.: A61K 48/00, C12N 15/864, A61P 43/00

(54) **Telomerase reverse transcriptase for protection against ageing**
Telomerase-Umkehrtranskriptase zum Schutz vor Alterung
Transcriptase inverse de la télomérase pour la protection contre le vieillissement

(43) Date of publication of application: 04.01.2012
(73) Proprietor: Fundación Centro Nacional De Investigaciones Oncológicas Carlos III, 28029 Madrid (ES); Universitat Autònoma de Barcelona, 08193 Bellaterra, Barcelona (ES)
(72) Inventor: Blasco Marhuenda, María Antonia, 28029 Madrid (ES); Bernardes, Bruno, 28029 Madrid (ES); Bosch Tubert, Fátima, 08193 Bellaterra - Barcelona (ES); Ayuso, Eduard, 08193, Bellaterra - Barcelona (ES)
(74) Representative: Elzaburu Marquez, Alberto

(56) References cited:
- WO-A2-99/35243
- TOMAS-LOBA ANTONIA ET AL: "Telomerase Reverse Transcriptase Delays Aging in Cancer-Resistant Mice", CELL, CELL PRESS, CAMBRIDGE, NA, US, vol. 135, no. 4, 1 November 2008 (2008-11-01), pages 609-622, XP002532531, ISSN: 0092-8674, DOI: DOI:10.1016/J.CELL.208.09.034
- WYLLIE F S ET AL: "Telomerase prevents the accelerated cell ageing of Werner syndrome fibroblasts", NATURE GENETICS, NATURE PUBLISHING GROUP, NEW YORK, US, vol. 24, 1 January 2000 (2000-01-01), pages 16-17, XP002188246, ISSN: 1061-4036, DOI: DOI:10.1038/71630
- DAGARAG MIRABELLE ET AL: "Genetic manipulation of telomerase in HIV-specific CD8+ T cells: enhanced antiviral functions accompany the increased proliferative potential and telomere length stabilization.", JOURNAL OF IMMUNOLOGY (BALTIMORE, MD. : 1950) 15 NOV 2004 LNKD- PUBMED:15528369, vol. 173, no. 10, 15 November 2004 (2004-11-15), pages 6303-6311, XP002624260, ISSN: 0022-1767
- BODNAR A G ET AL: "Extension of life-span by introduction of telomerase into normal human cells", 16 January 1998 (1998-01-16), SCIENCE, AMERICAN ASSOCIATION FOR THE ADVANCEMENT OF SCIENCE, WASHINGTON, DC; US, PAGE(S) 349 - 352, XP002902750, ISSN: 0036-8075 * page 349 * * abstract * * page 352, paragraph 1 *
- NG S S M ET AL: "A novel glioblastoma cancer gene therapy using AAV-mediated long-term expression of human TERT C-terminal polypeptide", CANCER GENE THERAPY, NORWALK, CT, US, vol. 14, no. 6, 1 June 2007 (2007-06-01), pages 561-572, XP002508171, ISSN: 0929-1903, DOI: DOI:10.1038/SJ.CGT.7701038 [retrieved on 2007-03-23]
- EMBO MOL MED vol. 4, 2012, pages 1 - 14
- EMBO MOL MED vol. 4, 2012, pages 685 - 687
- AGEING RESEARCH REVIEWS vol. 12, 2013, pages 310 - 315

### SEQUENCE LISTING

<110> FUNDACIÓN CENTRO NACIONAL DE INVESTIGACIONES ONCOLÓGICAS CARLOS III; UNIVERSITAT AUTONOMA DE BARCELONA
<120> Telomerase Reverse Transcriptase for Protection Against Ageing
<130> EP-865
<160> 22
<170> SeqWin99, version 1.02
<210> 1
   <211> 4018
   <212> DNA
   <213> Homo sapiens
<400> 1
   caggcagcgc tgcgtcctgc tgcgcacgtg ggaagccctg gccccggcca cccccgcgat   60
   gccgcgcgct ccccgctgcc gagccgtgcg ctccctgctg cgcagccact accgcgaggt  120
   gctgccgctg gccacgttcg tgcggcgcct ggggccccag ggctggcggc tggtgcagcg  180
   cggggacccg gcggctttcc gcgcgctggt ggcccagtgc ctggtgtgcg tgccctggga  240
   cgcacggccg ccccccgccg ccccctcctt ccgccaggtg tcctgcctga aggagctggt  300
   ggcccgagtg ctgcagaggc tgtgcgagcg cggcgcgaag aacgtgctgg ccttcggctt  360
   cgcgctgctg gacggggccc gcgggggccc ccccgaggcc ttcaccacca gcgtgcgcag  420
   ctacctgccc aacacggtga ccgacgcact gcgggggagc ggggcgtggg ggctgctgct  480
   gcgccgcgtg ggcgacgacg tgctggttca cctgctggca cgctgcgcgc tctttgtgct  540
   ggtggctccc agctgcgcct accaggtgtg cgggccgccg ctgtaccagc tcggcgctgc  600
   cactcaggcc cggcccccgc cacacgctag tggaccccga aggcgtctgg gatgcgaacg  660
   ggcctggaac catagcgtca gggaggccgg ggtccccctg ggcctgccag ccccgggtgc  720
   gaggaggcgc gggggcagtg ccagccgaag tctgccgttg cccaagaggc ccaggcgtgg  780
   cgctgcccct gagccggagc ggacgcccgt tgggcagggg tcctgggccc acccgggcag  840
   gacgcgtgga ccgagtgacc gtggtttctg tgtggtgtca cctgccagac ccgccgaaga  900
   agccacctct ttggagggtg cgctctctgg cacgcgccac tcccacccat ccgtgggccg  960
   ccagcaccac gcgggccccc catccacatc gcggccacca cgtccctggg acacgccttg 1020
   tcccccggtg tacgccgaga ccaagcactt cctctactcc tcaggcgaca aggagcagct 1080
   gcggccctcc ttcctactca gctctctgag gcccagcctg actggcgctc ggaggctcgt 1140
   ggagaccatc tttctgggtt ccaggccctg gatgccaggg actccccgca ggttgccccg 1200
   cctgccccag cgctactggc aaatgcggcc cctgtttctg gagctgcttg ggaaccacgc 1260
   gcagtgcccc tacggggtgc tcctcaagac gcactgcccg ctgcgagctg cggtcacccc 1320
   agcagccggt gtctgtgccc gggagaagcc ccagggctct gtggcggccc ccgaggagga 1380
   ggacacagac ccccgtcgcc tggtgcagct gctccgccag cacagcagcc cctggcaggt 1440
   gtacggcttc gtgcgggcct gcctgcgccg gctggtgccc ccaggcctct ggggctccag 1500
   gcacaacgaa cgccgcttcc tcaggaacac caagaagttc atctccctgg ggaagcatgc 1560
   caagctctcg ctgcaggagc tgacgtggaa gatgagcgtg cgggactgcg cttggctgcg 1620
   caggagccca ggggttggct gtgttccggc cgcagagcac cgtctgcgtg aggagatcct 1680
   ggccaagttc ctgcactggc tgatgagtgt gtacgtcgtc gagctgctca ggtctttctt 1740
   ttatgtcacg gagaccacgt ttcaaaagaa caggctcttt ttctaccgga agagtgtctg 1800
   gagcaagttg caaagcattg gaatcagaca gcacttgaag agggtgcagc tgcgggagct 1860
   gtcggaagca gaggtcaggc agcatcggga agccaggccc gccctgctga cgtccagact 1920
   ccgcttcatc cccaagcctg acggctgcgg gccgattgtg aacatggact acgtcgtggg 1980
   agccagaacg ttccgcagag aaaagagggc cgagcgtctc acctcgaggg tgaaggcact 2040
   gttcagcgtg ctcaactacg agcgggcgcg gcgccccggc ctcctgggcg cctctgtgct 2100
   gggcctggac gatatccaca gggcctggcg caccttcgtg ctgcgtgtgc gggcccagga 2160
   cccgccgcct gagctgtact ttgtcaaggt ggatgtgacg ggcgcgtacg acaccatccc 2220
   ccaggacagg ctcacggagg tcatcgccag catcatcaaa ccccagaaca cgtactgcgt 2280
   gcgtcggtat gccgtggtcc agaaggccgc ccatgggcac gtccgcaagg ccttcaagag 2340
   ccacgtctct accttgacag acctccagcc gtacatgcga cagttcgtgg ctcacctgca 2400
   ggagaccagc ccgctgaggg atgccgtcgt catcgagcag agctcctccc tgaatgaggc 2460
   cagcagtggc ctcttcgacg tcttcctacg cttcatgtgc caccacgccg tgcgcatcag   2520
   gggcaagtcc tacgtccagt gccaggggat cccgcagggc tccatcctct ccacgctgct   2580
   ctgcagcctg tgctacggcg acatggagaa caagctgttt gcggggattc ggcgggacgg   2640
   gctgctcctg cgtttggtgg atgatttctt gttggtgaca cctcacctca cccacgcgaa   2700
   aaccttcctc aggaccctgg tccgaggtgt ccctgagtat ggctgcgtgg tgaacttgcg   2760
   gaagacagtg gtgaacttcc ctgtagaaga cgaggccctg ggtggcacgg cttttgttca   2820
   gatgccggcc cacggcctat tcccctggtg cggcctgctg ctggataccc ggaccctgga   2880
   ggtgcagagc gactactcca gctatgcccg gacctccatc agagccagtc tcaccttcaa   2940
   ccgcggcttc aaggctggga ggaacatgcg tcgcaaactc tttggggtct tgcggctgaa   3000
   gtgtcacagc ctgtttctgg atttgcaggt gaacagcctc cagacggtgt gcaccaacat   3060
   ctacaagatc ctcctgctgc aggcgtacag gtttcacgca tgtgtgctgc agctcccatt   3120
   tcatcagcaa gtttggaaga accccacatt tttcctgcgc gtcatctctg acacggcctc   3180
   cctctgctac tccatcctga aagccaagaa cgcagggatg tcgctggggg ccaagggcgc   3240
   cgccggccct ctgccctccg aggccgtgca gtggctgtgc caccaagcat tcctgctcaa   3300
   gctgactcga caccgtgtca cctacgtgcc actcctgggg tcactcagga cagcccagac   3360
   gcagctgagt cggaagctcc cggggacgac gctgactgcc ctggaggccg cagccaaccc   3420
   ggcactgccc tcagacttca agaccatcct ggactgatgg ccacccgccc acagccaggc   3480
   cgagagcaga caccagcagc cctgtcacgc cgggctctac gtcccaggga gggaggggcg   3540
   gcccacaccc aggcccgcac cgctgggagt ctgaggcctg agtgagtgtt tggccgaggc   3600
   ctgcatgtcc ggctgaaggc tgagtgtccg gctgaggcct gagcgagtgt ccagccaagg   3660
   gctgagtgtc cagcacacct gccgtcttca cttccccaca ggctggcgct cggctccacc   3720
   ccagggccag cttttcctca ccaggagccc ggcttccact ccccacatag gaatagtcca   3780
   tccccagatt cgccattgtt cacccctcgc cctgccctcc tttgccttcc acccccacca   3840
   tccaggtgga gaccctgaga aggaccctgg gagctctggg aatttggagt gaccaaaggt   3900
   gtgccctgta cacaggcgag gaccctgcac ctggatgggg gtccctgtgg gtcaaattgg   3960
   ggggaggtgc tgtgggagta aaatactgaa tatatgagtt tttcagtttt gaaaaaaa      4018
<210> 2
   <211> 1132
   <212> PRT
   <213> Homo sapiens
<400> 2
<210> 3
   <211> 3982
   <212> DNA
   <213> Homo sapiens
<400> 3
   caggcagcgc tgcgtcctgc tgcgcacgtg ggaagccctg gccccggcca cccccgcgat   60
   gccgcgcgct ccccgctgcc gagccgtgcg ctccctgctg cgcagccact accgcgaggt  120
   gctgccgctg gccacgttcg tgcggcgcct ggggccccag ggctggcggc tggtgcagcg  180
   cggggacccg gcggctttcc gcgcgctggt ggcccagtgc ctggtgtgcg tgccctggga  240
   cgcacggccg ccccccgccg cccctccctt ccgccaggtg tcctgcctga aggagctggt  300
   ggcccgagtg ctgcagaggc tgtgcgagcg cggcgcgaag aacgtgctgg ccttcggctt  360
   cgcgctgctg gacggggccc gcgggggccc ccccgaggcc ttcaccacca gcgtgcgcag  420
   ctacctgccc aacacggtga ccgacgcact gcgggggagc ggggcgtggg ggctgctgct  480
   gcgccgcgtg ggcgacgacg tgctggttca cctgctggca cgctgcgcgc tctttgtgct  540
   ggtggctccc agctgcgcct accaggtgtg cgggccgccg ctgtaccagc tcggcgctgc  600
   cactcaggcc cggcccccgc cacacgctag tggaccccga aggcgtctgg gatgcgaacg  660
   ggcctggaac catagcgtca gggaggccgg ggtccccctg ggcctgccag ccccgggtgc  720
   gaggaggcgc gggggcagtg ccagccgaag tctgccgttg cccaagaggc ccaggcgtgg  780
   cgctgcccct gagccggagc ggacgcccgt tgggcagggg tcctgggccc acccgggcag  840
   gacgcgtgga ccgagtgacc gtggtttctg tgtggtgtca cctgccagac ccgccgaaga  900
   agccacctct ttggagggtg cgctctctgg cacgcgccac tcccacccat ccgtgggccg  960
   ccagcaccac gcgggccccc catccacatc gcggccacca cgtccctggg acacgccttg 1020
   tcccccggtg tacgccgaga ccaagcactt cctctactcc tcaggcgaca aggagcagct 1080
   gcggccctcc ttcctactca gctctctgag gcccagcctg actggcgctc ggaggctcgt 1140
   ggagaccatc tttctgggtt ccaggccctg gatgccaggg actccccgca ggttgccccg 1200
   cctgccccag cgctactggc aaatgcggcc cctgtttctg gagctgcttg ggaaccacgc 1260
   gcagtgcccc tacggggtgc tcctcaagac gcactgcccg ctgcgagctg cggtcacccc 1320
   agcagccggt gtctgtgccc gggagaagcc ccagggctct gtggcggccc ccgaggagga 1380
   ggacacagac ccccgtcgcc tggtgcagct gctccgccag cacagcagcc cctggcaggt 1440
   gtacggcttc gtgcgggcct gcctgcgccg gctggtgccc ccaggcctct ggggctccag 1500
   gcacaacgaa cgccgcttcc tcaggaacac caagaagttc atctccctgg ggaagcatgc 1560
   caagctctcg ctgcaggagc tgacgtggaa gatgagcgtg cgggactgcg cttggctgcg 1620
   caggagccca ggggttggct gtgttccggc cgcagagcac cgtctgcgtg aggagatcct 1680
   ggccaagttc ctgcactggc tgatgagtgt gtacgtcgtc gagctgctca ggtctttctt 1740
   ttatgtcacg gagaccacgt ttcaaaagaa caggctcttt ttctaccgga agagtgtctg 1800
   gagcaagttg caaagcattg gaatcagaca gcacttgaag agggtgcagc tgcgggagct 1860
   gtcggaagca gaggtcaggc agcatcggga agccaggccc gccctgctga cgtccagact 1920
   ccgcttcatc cccaagcctg acgggctgcg gccgattgtg aacatggact acgtcgtggg 1980
   agccagaacg ttccgcagag aaaagagggc cgagcgtctc acctcgaggg tgaaggcact 2040
   gttcagcgtg ctcaactacg agcgggcgcg gcgccccggc ctcctgggcg cctctgtgct 2100
   gggcctggac gatatccaca gggcctggcg caccttcgtg ctgcgtgtgc gggcccagga 2160
   cccgccgcct gagctgtact ttgtcaagga caggctcacg gaggtcatcg ccagcatcat 2220
   caaaccccag aacacgtact gcgtgcgtcg gtatgccgtg gtccagaagg ccgcccatgg 2280
   gcacgtccgc aaggccttca agagccacgt ctctaccttg acagacctcc agccgtacat 2340
   gcgacagttc gtggctcacc tgcaggagac cagcccgctg agggatgccg tcgtcatcga 2400
   gcagagctcc tccctgaatg aggccagcag tggcctcttc gacgtcttcc tacgcttcat 2460
   gtgccaccac gccgtgcgca tcaggggcaa gtcctacgtc cagtgccagg ggatcccgca 2520
   gggctccatc ctctccacgc tgctctgcag cctgtgctac ggcgacatgg agaacaagct 2580
   gtttgcgggg attcggcggg acgggctgct cctgcgtttg gtggatgatt tcttgttggt 2640
   gacacctcac ctcacccacg cgaaaacctt cctcaggacc ctggtccgag gtgtccctga 2700
   gtatggctgc gtggtgaact tgcggaagac agtggtgaac ttccctgtag aagacgaggc 2760
   cctgggtggc acggcttttg ttcagatgcc ggcccacggc ctattcccct ggtgcggcct   2820
   gctgctggat acccggaccc tggaggtgca gagcgactac tccagctatg cccggacctc   2880
   catcagagcc agtctcacct tcaaccgcgg cttcaaggct gggaggaaca tgcgtcgcaa   2940
   actctttggg gtcttgcggc tgaagtgtca cagcctgttt ctggatttgc aggtgaacag   3000
   cctccagacg gtgtgcacca acatctacaa gatcctcctg ctgcaggcgt acaggtttca   3060
   cgcatgtgtg ctgcagctcc catttcatca gcaagtttgg aagaacccca catttttcct   3120
   gcgcgtcatc tctgacacgg cctccctctg ctactccatc ctgaaagcca agaacgcagg   3180
   gatgtcgctg gggGccaagg gcgccgccgg ccctctgccc tccgaggccg tgcagtggct   3240
   gtgccaccaa gcattcctgc tcaagctgac tcgacaccgt gtcacctacg tgccactcct   3300
   ggggtcactc aggacagccc agacgcagct gagtcggaag ctcccgggga cgacgctgac   3360
   tgccctggag gccgcagcca acccggcact gccctcagac ttcaagacca tcctggactg   3420
   atggccaccc gcccacagcc aggccgagag cagacaccag cagccctgtc acgccgggct   3480
   ctacgtccca gggagggagg ggcggcccac acccaggccc gcaccgctgg gagtctgagg   3540
   cctgagtgag tgtttggccg aggcctgcat gtccggctga aggctgagtg tccggctgag   3600
   gcctgagcga gtgtccagcc aagggctgag tgtccagcac acctgccgtc ttcacttccc   3660
   cacaggctgg cgctcggctc caccccaggg ccagcttttc ctcaccagga gcccggcttc   3720
   cactccccac ataggaatag tccatcccca gattcgccat tgttcacccc tcgccctgcc   3780
   ctcctttgcc ttccaccccc accatccagg tggagaccct gagaaggacc ctgggagctc   3840
   tgggaatttg gagtgaccaa aggtgtgccc tgtacacagg cgaggaccct gcacctggat   3900
   gggggtccct gtgggtcaaa ttggggggag gtgctgtggg agtaaaatac tgaatatatg   3960
   agtttttcag ttttgaaaaa aa                                            3982
<210> 4
   <211> 1120
   <212> PRT
   <213> Homo sapiens
<400> 4
   Met Pro Arg Ala Pro Arg Cys Arg Ala Val Arg Ser Leu Leu Arg Ser
   1               5                   10                  15
   His Tyr Arg Glu Val Leu Pro Leu Ala Thr Phe Val Arg Arg Leu Gly
               20                  25                  30
   Pro Gln Gly Trp Arg Leu Val Gln Arg Gly Asp Pro Ala Ala Phe Arg
           35                  40                  45
   Ala Leu Val Ala Gln Cys Leu Val Cys Val Pro Trp Asp Ala Arg Pro
       50                  55                  60
   Pro Pro Ala Ala Pro Ser Phe Arg Gln Val Ser Cys Leu Lys Glu Leu
   65                  70                  75                  80
   Val Ala Arg Val Leu Gln Arg Leu Cys Glu Arg Gly Ala Lys Asn Val
                   85                  90                  95
   Leu Ala Phe Gly Phe Ala Leu Leu Asp Gly Ala Arg Gly Gly Pro Pro
               100                 105                 110
   Glu Ala Phe Thr Thr Ser Val Arg Ser Tyr Leu Pro Asn Thr Val Thr
           115                 120                 125
   Asp Ala Leu Arg Gly Ser Gly Ala Trp Gly Leu Leu Leu Arg Arg Val
       130                 135                 140
   Gly Asp Asp Val Leu Val His Leu Leu Ala Arg Cys Ala Leu Phe Val
   145                 150                 155                 160
   Leu Val Ala Pro Ser Cys Ala Tyr Gln Val Cys Gly Pro Pro Leu Tyr
   165 170 175
   Gln Leu Gly Ala Ala Thr Gln Ala Arg Pro Pro Pro His Ala Ser Gly
   180 185 190
   Pro Arg Arg Arg Leu Gly Cys Glu Arg Ala Trp Asn His Ser Val Arg
   195 200 205
   Glu Ala Gly Val Pro Leu Gly Leu Pro Ala Pro Gly Ala Arg Arg Arg
   210 215 220
   Gly Gly Ser Ala Ser Arg Ser Leu Pro Leu Pro Lys Arg Pro Arg Arg
   225 230 235 240
   Gly Ala Ala Pro Glu Pro Glu Arg Thr Pro Val Gly Gln Gly Ser Trp
   245 250 255
   Ala His Pro Gly Arg Thr Arg Gly Pro Ser Asp Arg Gly Phe Cys Val
   260 265 270
   Val Ser Pro Ala Arg Pro Ala Glu Glu Ala Thr Ser Leu Glu Gly Ala
   275 280 285
   Leu Ser Gly Thr Arg His Ser His Pro Ser Val Gly Arg Gln His His
   290 295 300
   Ala Gly Pro Pro Ser Thr Ser Arg Pro Pro Arg Pro Trp Asp Thr Pro
   305 310 315 320
   Cys Pro Pro Val Tyr Ala Glu Thr Lys His Phe Leu Tyr Ser Ser Gly
   325 330 335
   Asp Lys Glu Gln Leu Arg Pro Ser Phe Leu Leu Ser Ser Leu Arg Pro
   340 345 350
   Ser Leu Thr Gly Ala Arg Arg Leu Val Glu Thr Ile Phe Leu Gly Ser
   355 360 365
   Arg Pro Trp Met Pro Gly Thr Pro Arg Arg Leu Pro Arg Leu Pro Gln
   370 375 380
   Arg Tyr Trp Gln Met Arg Pro Leu Phe Leu Glu Leu Leu Gly Asn His
   385 390 395 400
   Ala Gln Cys Pro Tyr Gly Val Leu Leu Lys Thr His Cys Pro Leu Arg
   405 410 415
   Ala Ala Val Thr Pro Ala Ala Gly Val Cys Ala Arg Glu Lys Pro Gln
   420 425 430
   Gly Ser Val Ala Ala Pro Glu Glu Glu Asp Thr Asp Pro Arg Arg Leu
   435 440 445
   Val Gln Leu Leu Arg Gln His Ser Ser Pro Trp Gln Val Tyr Gly Phe
   450 455 460
   Val Arg Ala Cys Leu Arg Arg Leu Val Pro Pro Gly Leu Trp Gly Ser
   465 470 475 480
   Arg His Asn Glu Arg Arg Phe Leu Arg Asn Thr Lys Lys Phe Ile Ser
                   485                 490                 495
   Leu Gly Lys His Ala Lys Leu Ser Leu Gln Glu Leu Thr Trp Lys Met
               500                 505                 510
   Ser Val Arg Asp Cys Ala Trp Leu Arg Arg Ser Pro Gly Val Gly Cys
           515                 520                 525
   Val Pro Ala Ala Glu His Arg Leu Arg Glu Glu Ile Leu Ala Lys Phe
       530                 535                 540
   Leu His Trp Leu Met Ser Val Tyr Val Val Glu Leu Leu Arg Ser Phe
   545                 550                 555                 560
   Phe Tyr Val Thr Glu Thr Thr Phe Gln Lys Asn Arg Leu Phe Phe Tyr
                   565                 570                 575
   Arg Lys Ser Val Trp Ser Lys Leu Gln Ser Ile Gly Ile Arg Gln His
               580                 585                 590
   Leu Lys Arg Val Gln Leu Arg Glu Leu Ser Glu Ala Glu Val Arg Gln
           595                 600                 605
   His Arg Glu Ala Arg Pro Ala Leu Leu Thr Ser Arg Leu Arg Phe Ile
       610                 615                 620
   Pro Lys Pro Asp Gly Leu Arg Pro Ile Val Asn Met Asp Tyr Val Val
   625                 630                 635                 640
   Gly Ala Arg Thr Phe Arg Arg Glu Lys Arg Ala Glu Arg Leu Thr Ser
                   645                 650                 655
   Arg Val Lys Ala Leu Phe Ser Val Leu Asn Tyr Glu Arg Ala Arg Arg
               660                 665                 670
   Pro Gly Leu Leu Gly Ala Ser Val Leu Gly Leu Asp Asp Ile His Arg
           675                 680                 685
   Ala Trp Arg Thr Phe Val Leu Arg Val Arg Ala Gln Asp Pro Pro Pro
       690                 695                 700
   Glu Leu Tyr Phe Val Lys Asp Arg Leu Thr Glu Val Ile Ala Ser Ile
   705                 710                 715                 720
   Ile Lys Pro Gln Asn Thr Tyr Cys Val Arg Arg Tyr Ala Val Val Gln
                   725                 730                 735
   Lys Ala Ala His Gly His Val Arg Lys Ala Phe Lys Ser His Val Ser
               740                 745                 750
   Thr Leu Thr Asp Leu Gln Pro Tyr Met Arg Gln Phe Val Ala His Leu
           755                 760                 765
   Gln Glu Thr Ser Pro Leu Arg Asp Ala Val Val Ile Glu Gln Ser Ser
       770                 775                 780
   Ser Leu Asn Glu Ala Ser Ser Gly Leu Phe Asp Val Phe Leu Arg Phe
   785                 790                 795                 800
   Met Cys His His Ala Val Arg Ile Arg Gly Lys Ser Tyr Val Gln Cys
   805 810 815
   Gln Gly Ile Pro Gln Gly Ser Ile Leu Ser Thr Leu Leu Cys Ser Leu
   820 825 830
   Cys Tyr Gly Asp Met Glu Asn Lys Leu Phe Ala Gly Ile Arg Arg Asp
   835 840 845
   Gly Leu Leu Leu Arg Leu Val Asp Asp Phe Leu Leu Val Thr Pro His
   850 855 860
   Leu Thr His Ala Lys Thr Phe Leu Arg Thr Leu Val Arg Gly Val Pro
   865 870 875 880
   Glu Tyr Gly Cys Val Val Asn Leu Arg Lys Thr Val Val Asn Phe Pro
   885 890 895
   Val Glu Asp Glu Ala Leu Gly Gly Thr Ala Phe Val Gln Met Pro Ala
   900 905 910
   His Gly Leu Phe Pro Trp Cys Gly Leu Leu Leu Asp Thr Arg Thr Leu
   915 920 925
   Glu Val Gln Ser Asp Tyr Ser Ser Tyr Ala Arg Thr Ser Ile Arg Ala
   930 935 940
   Ser Leu Thr Phe Asn Arg Gly Phe Lys Ala Gly Arg Asn Met Arg Arg
   945 950 955 960
   Lys Leu Phe Gly Val Leu Arg Leu Lys Cys His Ser Leu Phe Leu Asp
   965 970 975
   Leu Gln Val Asn Ser Leu Gln Thr Val Cys Thr Asn Ile Tyr Lys Ile
   980 985 990
   Leu Leu Leu Gln Ala Tyr Arg Phe His Ala Cys Val Leu Gln Leu Pro
   995 1000 1005
   Phe His Gln Gln Val Trp Lys Asn Pro Thr Phe Phe Leu Arg Val Ile
   1010 1015 1020
   Ser Asp Thr Ala Ser Leu Cys Tyr Ser Ile Leu Lys Ala Lys Asn Ala
   1025 1030 1035 1040
   Gly Met Ser Leu Gly Ala Lys Gly Ala Ala Gly Pro Leu Pro Ser Glu
   1045 1050 1055
   Ala Val Gln Trp Leu Cys His Gln Ala Phe Leu Leu Lys Leu Thr Arg
   1060 1065 1070
   His Arg Val Thr Tyr Val Pro Leu Leu Gly Ser Leu Arg Thr Ala Gln
   1075 1080 1085
   Thr Gln Leu Ser Arg Lys Leu Pro Gly Thr Thr Leu Thr Ala Leu Glu
   1090 1095 1100
   Ala Ala Ala Asn Pro Ala Leu Pro Ser Asp Phe Lys Thr Ile Leu Asp
   1105 1110 1115 1120
<210> 5
   <211> 3426
   <212> DNA
   <213> Mus musculus
<400> 5
   gtgggaggcc catcccggcc ttgagcacaa tgacccgcgc tcctcgttgc cccgcggtgc   60
   gctctctgct gcgcagccga taccgggagg tgtggccgct ggcaaccttt gtgcggcgcc  120
   tggggcccga gggcaggcgg cttgtgcaac ccggggaccc gaagatctac cgcactttgg  180
   ttgcccaatg cctagtgtgc atgcactggg gctcacagcc tccacctgcc gacctttcct  240
   tccaccaggt gtcatccctg aaagagctgg tggccagggt tgtgcagaga ctctgcgagc  300
   gcaacgagag aaacgtgctg gcttttggct ttgagctgct taacgaggcc agaggcgggc  360
   ctcccatggc cttcactagt agcgtgcgta gctacttgcc caacactgtt attgagaccc  420
   tgcgtgtcag tggtgcatgg atgctactgt tgagccgagt gggcgacgac ctgctggtct  480
   acctgctggc acactgtgct ctttatcttc tggtgccccc cagctgtgcc taccaggtgt  540
   gtgggtctcc cctgtaccaa atttgtgcca ccacggatat ctggccctct gtgtccgcta  600
   gttacaggcc cacccgaccc gtgggcagga atttcactaa ccttaggttc ttacaacaga  660
   tcaagagcag tagtcgccag gaagcaccga aacccctggc cttgccatct cgaggtacaa  720
   agaggcatct gagtctcacc agtacaagtg tgccttcagc taagaaggcc agatgctatc  780
   ctgtcccgag agtggaggag ggaccccaca ggcaggtgct accaacccca tcaggcaaat  840
   catgggtgcc aagtcctgct cggtcccccg aggtgcctac tgcagagaaa gatttgtctt  900
   ctaaaggaaa ggtgtctgac ctgagtctct ctgggtcggt gtgctgtaaa cacaagccca  960
   gctccacatc tctgctgtca ccaccccgcc aaaatgcctt tcagctcagg ccatttattg 1020
   agaccagaca tttcctttac tccaggggag atggccaaga gcgtctaaac ccctcattcc  1080
   tactcagcaa cctccagcct aacttgactg gggccaggag actggtggag atcatctttc  1140
   tgggctcaag gcctaggaca tcaggaccac tctgcaggac acaccgtcta tcgcgtcgat 1200
   actggcagat gcggcccctg ttccaacagc tgctggtgaa ccatgcagag tgccaatatg 1260
   tcagactcct caggtcacat tgcaggtttc gaacagcaaa ccaacaggtg acagatgcct 1320
   tgaacaccag cccaccgcac ctcatggatt tgctccgcct gcacagcagt ccctggcagg 1380
   tatatggttt tcttcgggcc tgtctctgca aggtggtgtc tgctagtctc tggggtacca 1440
   ggcacaatga gcgccgcttc tttaagaact taaagaagtt catctcgttg gggaaatacg  1500
   gcaagctatc actgcaggaa ctgatgtgga agatgaaagt agaggattgc cactggctcc 1560
   gcagcagccc ggggaaggac cgtgtccccg ctgcagagca ccgtctgagg gagaggatcc 1620
   tggctacgtt cctgttctgg ctgatggaca catacgtggt acagctgctt aggtcattct 1680
   tttacatcac agagagcaca ttccagaaga acaggctctt cttctaccgt aagagtgtgt 1740
   ggagcaagct gcagagcatt ggagtcaggc aacaccttga gagagtgcgg ctacgggagc 1800
   tgtcacaaga ggaggtcagg catcaccagg acacctggct agccatgccc atctgcagac 1860
   tgcgcttcat ccccaagccc aacggcctgc ggcccattgt gaacatgagt tatagcatgg 1920
   gtaccagagc tttgggcaga aggaagcagg cccagcattt cacccagcgt ctcaagactc 1980
   tcttcagcat gctcaactat gagcggacaa aacatcctca ccttatgggg tcttctgtac 2040
   tgggtatgaa tgacatctac aggacctggc gggcctttgt gctgcgtgtg cgtgctctgg 2100
   accagacacc caggatgtac tttgttaagg cagatgtgac cggggcctat gatgccatcc 2160
   cccagggtaa gctggtggag gttgttgcca atatgatcag gcactcggag agcacgtact 2220
   gtatccgcca gtatgcagtg gtccggagag atagccaagg ccaagtccac aagtccttta 2280
   ggagacaggt caccaccctc tctgacctcc agccatacat gggccagttc cttaagcatc 2340
   tgcaggattc agatgccagt gcactgagga actccgttgt catcgagcag agcatctcta 2400
   tgaatgagag cagcagcagc ctgtttgact tcttcctgca cttcctgcgt cacagtgtcg 2460
   taaagattgg tgacaggtgc tatacgcagt gccagggcat cccccagggc tccagcctat 2520
   ccaccctgct ctgcagtctg tgtttcggag acatggagaa caagctgttt gctgaggtgc 2580
   agcgggatgg gttgctttta cgttttgttg atgactttct gttggtgacg cctcacttgg 2640
   accaagcaaa aaccttcctc agcaccctgg tccatggcgt tcctgagtat gggtgcatga 2700
   taaacttgca gaagacagtg gtgaacttcc ctgtggagcc tggtaccctg ggtggtgcag 2760
   ctccatacca gctgcctgct cactgcctgt ttccctggtg tggcttgctg ctggacactc 2820
   agactttgga ggtgttctgt gactactcag gttatgccca gacctcaatt aagacgagcc 2880
   tcaccttcca gagtgtcttc aaagctggga agaccatgcg gaacaagctc ctgtcggtct 2940
   tgcggttgaa gtgtcacggt ctatttctag acttgcaggt gaacagcctc cagacagtct 3000
   gcatcaatat atacaagatc ttcctgcttc aggcctacag gttccatgca tgtgtgattc 3060
   agcttccctt tgaccagcgt gttaggaaga acctcacatt ctttctgggc atcatctcca 3120
   gccaagcatc ctgctgctat gctatcctga aggtcaagaa tccaggaatg acactaaagg 3180
   cctctggctc ctttcctcct gaagccgcac attggctctg ctaccaggcc ttcctgctca 3240
   agctggctgc tcattctgtc atctacaaat gtctcctggg acctctgagg acagcccaaa 3300
   aactgctgtg ccggaagctc ccagaggcga caatgaccat ccttaaagct gcagctgacc 3360
   cagccctaag cacagacttt cagaccattt tggactaacc ctgtctcctt ccgctagatg 3420
   aacatg                                                             3426
<210> 6
   <211> 1122
   <212> PRT
   <213> Mus musculus
<400> 6
   Met Thr Arg Ala Pro Arg Cys Pro Ala Val Arg Ser Leu Leu Arg Ser
   1               5                   10                  15
   Arg Tyr Arg Glu Val Trp Pro Leu Ala Thr Phe Val Arg Arg Leu Gly
               20                  25                  30
   Pro Glu Gly Arg Arg Leu Val Gln Pro Gly Asp Pro Lys Ile Tyr Arg
           35                  40                  45
   Thr Leu Val Ala Gln Cys Leu Val Cys Met His Trp Gly Ser Gln Pro
       50                  55                  60
   Pro Pro Ala Asp Leu Ser Phe His Gln Val Ser Ser Leu Lys Glu Leu
   65                  70                  75                  80
   Val Ala Arg Val Val Gln Arg Leu Cys Glu Arg Asn Glu Arg Asn Val
                   85                  90                  95
   Leu Ala Phe Gly Phe Glu Leu Leu Asn Glu Ala Arg Gly Gly Pro Pro
               100                 105                 110
   Met Ala Phe Thr Ser Ser Val Arg Ser Tyr Leu Pro Asn Thr Val Ile
           115                 120                 125
   Glu Thr Leu Arg Val Ser Gly Ala Trp Met Leu Leu Leu Ser Arg Val
       130                 135                 140
   Gly Asp Asp Leu Leu Val Tyr Leu Leu Ala His Cys Ala Leu Tyr Leu
   145                 150                 155                 160
   Leu Val Pro Pro Ser Cys Ala Tyr Gln Val Cys Gly Ser Pro Leu Tyr
                   165                 170                 175
   Gln Ile Cys Ala Thr Thr Asp Ile Trp Pro Ser Val Ser Ala Ser Tyr
               180                 185                 190
   Arg Pro Thr Arg Pro Val Gly Arg Asn Phe Thr Asn Leu Arg Phe Leu
           195                 200                 205
   Gln Gln Ile Lys Ser Ser Ser Arg Gln Glu Ala Pro Lys Pro Leu Ala
       210                 215                 220
   Leu Pro Ser Arg Gly Thr Lys Arg His Leu Ser Leu Thr Ser Thr Ser
   225                 230                 235                 240
   Val Pro Ser Ala Lys Lys Ala Arg Cys Tyr Pro Val Pro Arg Val Glu
                   245                 250                 255
   Glu Gly Pro His Arg Gln Val Leu Pro Thr Pro Ser Gly Lys Ser Trp
               260                 265                 270
   Val Pro Ser Pro Ala Arg Ser Pro Glu Val Pro Thr Ala Glu Lys Asp
           275                 280                 285
   Leu Ser Ser Lys Gly Lys Val Ser Asp Leu Ser Leu Ser Gly Ser Val
       290                 295                 300
   Cys Cys Lys His Lys Pro Ser Ser Thr Ser Leu Leu Ser Pro Pro Arg
   305                 310                 315                 320
   Gln Asn Ala Phe Gln Leu Arg Pro Phe Ile Glu Thr Arg His Phe Leu
                   325                 330                 335
   Tyr Ser Arg Gly Asp Gly Gln Glu Arg Leu Asn Pro Ser Phe Leu Leu
               340                 345                 350
   Ser Asn Leu Gln Pro Asn Leu Thr Gly Ala Arg Arg Leu Val Glu Ile
           355                 360                 365
   Ile Phe Leu Gly Ser Arg Pro Arg Thr Ser Gly Pro Leu Cys Arg Thr
       370                 375                 380
   His Arg Leu Ser Arg Arg Tyr Trp Gln Met Arg Pro Leu Phe Gln Gln
   385                 390                 395                 400
   Leu Leu Val Asn His Ala Glu Cys Gln Tyr Val Arg Leu Leu Arg Ser
                   405                 410                 415
   His Cys Arg Phe Arg Thr Ala Asn Gln Gln Val Thr Asp Ala Leu Asn
               420                 425                 430
   Thr Ser Pro Pro His Leu Met Asp Leu Leu Arg Leu His Ser Ser Pro
           435                 440                 445
   Trp Gln Val Tyr Gly Phe Leu Arg Ala Cys Leu Cys Lys Val Val Ser
       450                 455                 460
   Ala Ser Leu Trp Gly Thr Arg His Asn Glu Arg Arg Phe Phe Lys Asn
   465                 470                 475                 480
   Leu Lys Lys Phe Ile Ser Leu Gly Lys Tyr Gly Lys Leu Ser Leu Gln
                   485                 490                 495
   Glu Leu Met Trp Lys Met Lys Val Glu Asp Cys His Trp Leu Arg Ser
               500                 505                 510
   Ser Pro Gly Lys Asp Arg Val Pro Ala Ala Glu His Arg Leu Arg Glu
           515                 520                 525
   Arg Ile Leu Ala Thr Phe Leu Phe Trp Leu Met Asp Thr Tyr Val Val
       530                 535                 540
   Gln Leu Leu Arg Ser Phe Phe Tyr Ile Thr Glu Ser Thr Phe Gln Lys
   545                 550                 555                 560
   Asn Arg Leu Phe Phe Tyr Arg Lys Ser Val Trp Ser Lys Leu Gln Ser
                   565                 570                 575
   Ile Gly Val Arg Gln His Leu Glu Arg Val Arg Leu Arg Glu Leu Ser
               580                 585                 590
   Gln Glu Glu Val Arg His His Gln Asp Thr Trp Leu Ala Met Pro Ile
           595                 600                 605
   Cys Arg Leu Arg Phe Ile Pro Lys Pro Asn Gly Leu Arg Pro Ile Val
       610                 615                 620
   Asn Met Ser Tyr Ser Met Gly Thr Arg Ala Leu Gly Arg Arg Lys Gln
   625                 630                 635                 640
   Ala Gln His Phe Thr Gln Arg Leu Lys Thr Leu Phe Ser Met Leu Asn
                   645                 650                 655
   Tyr Glu Arg Thr Lys His Pro His Leu Met Gly Ser Ser Val Leu Gly
               660                 665                 670
   Met Asn Asp Ile Tyr Arg Thr Trp Arg Ala Phe Val Leu Arg Val Arg
           675                 680                 685
   Ala Leu Asp Gln Thr Pro Arg Met Tyr Phe Val Lys Ala Asp Val Thr
       690                 695                 700
   Gly Ala Tyr Asp Ala Ile Pro Gln Gly Lys Leu Val Glu Val Val Ala
   705                 710                 715                 720
   Asn Met Ile Arg His Ser Glu Ser Thr Tyr Cys Ile Arg Gln Tyr Ala
                   725                 730                 735
   Val Val Arg Arg Asp Ser Gln Gly Gln Val His Lys Ser Phe Arg Arg
               740                 745                 750
   Gln Val Thr Thr Leu Ser Asp Leu Gln Pro Tyr Met Gly Gln Phe Leu
           755                 760                 765
   Lys His Leu Gln Asp Ser Asp Ala Ser Ala Leu Arg Asn Ser Val Val
       770                 775                 780
   Ile Glu Gln Ser Ile Ser Met Asn Glu Ser Ser Ser Ser Leu Phe Asp
   785                 790                 795                 800
   Phe Phe Leu His Phe Leu Arg His Ser Val Val Lys Ile Gly Asp Arg
                   805                 810                 815
   Cys Tyr Thr Gln Cys Gln Gly Ile Pro Gln Gly Ser Ser Leu Ser Thr
               820                 825                 830
   Leu Leu Cys Ser Leu Cys Phe Gly Asp Met Glu Asn Lys Leu Phe Ala
           835                 840                 845
   Glu Val Gln Arg Asp Gly Leu Leu Leu Arg Phe Val Asp Asp Phe Leu
       850                 855                 860
   Leu Val Thr Pro His Leu Asp Gln Ala Lys Thr Phe Leu Ser Thr Leu
   865                 870                 875                 880
   Val His Gly Val Pro Glu Tyr Gly Cys Met Ile Asn Leu Gln Lys Thr
                   885                 890                 895
   Val Val Asn Phe Pro Val Glu Pro Gly Thr Leu Gly Gly Ala Ala Pro
               900                 905                 910
   Tyr Gln Leu Pro Ala His Cys Leu Phe Pro Trp Cys Gly Leu Leu Leu
           915                 920                 925
   Asp Thr Gln Thr Leu Glu Val Phe Cys Asp Tyr Ser Gly Tyr Ala Gln
       930                 935                 940
   Thr Ser Ile Lys Thr Ser Leu Thr Phe Gln Ser Val Phe Lys Ala Gly
   945                 950                 955                 960
   Lys Thr Met Arg Asn Lys Leu Leu Ser Val Leu Arg Leu Lys Cys His
                   965                 970                 975
   Gly Leu Phe Leu Asp Leu Gln Val Asn Ser Leu Gln Thr Val Cys Ile
               980                 985                 990
   Asn Ile Tyr Lys Ile Phe Leu Leu Gln Ala Tyr Arg Phe His Ala Cys
           995                 1000                1005
   Val Ile Gln Leu Pro Phe Asp Gln Arg Val Arg Lys Asn Leu Thr Phe
       1010                1015                1020
   Phe Leu Gly Ile Ile Ser Ser Gln Ala Ser Cys Cys Tyr Ala Ile Leu
   1025                1030                1035                1040
   Lys Val Lys Asn Pro Gly Met Thr Leu Lys Ala Ser Gly Ser Phe Pro
                   1045                1050                1055
   Pro Glu Ala Ala His Trp Leu Cys Tyr Gln Ala Phe Leu Leu Lys Leu
               1060                1065                1070
   Ala Ala His Ser Val Ile Tyr Lys Cys Leu Leu Gly Pro Leu Arg Thr
           1075                1080                1085
   Ala Gln Lys Leu Leu Cys Arg Lys Leu Pro Glu Ala Thr Met Thr Ile
       1090                1095                1100
   Leu Lys Ala Ala Ala Asp Pro Ala Leu Ser Thr Asp Phe Gln Thr Ile
   1105                1110                1115                1120
   Leu Asp
<210> 7
   <211> 21
   <212> DNA
   <213> Artificial Sequence
<220>
   <223> Primer
<400> 7
   ggcaccacac cttctacaat g
<210> 8
   <211> 18
   <212> DNA
   <213> Artificial Sequence
<220>
   <223> Primer
<400> 8
   gtggtggtga agctgtag 18
<210> 9
   <211> 18
   <212> DNA
   <213> Artificial Sequence
<220>
   <223> Primer
<400> 9
   ggattgccac tggctccg 18
<210> 10
   <211> 21
   <212> DNA
   <213> Artificial Sequence
<220>
   <223> Primer
<400> 10
   tgcctgacct cctcttgtga c 21
<210> 11
   <211> 20
   <212> DNA
   <213> Artificial Sequence
<220>
   <223> Primer
<400> 11
   cgtaccccga ttcaggtgat 20
<210> 12
   <211> 22
   <212> DNA
   <213> Artificial Sequence
<220>
   <223> Primer
<400> 12
   ttgagcagaa gagctgctac gt 22
<210> 13
   <211> 21
   <212> DNA
   <213> Artificial Sequence
<220>
   <223> Primer
<400> 13
   ggcaaagtgg agaggatcga c 21
<210> 14
   <211> 18
   <212> DNA
   <213> Artificial Sequence
<220>
   <223> Primer
<400> 14
   tcgtggctgt tgcgtagg 18
<210> 15
   <211> 19
   <212> DNA
   <213> Artificial Sequence
<220>
   <223> Primer
<400> 15
   tgcgccctcc gtatcttac 19
<210> 16
   <211> 22
   <212> DNA
   <213> Artificial Sequence
<220>
   <223> Primer
<400> 16
   atcttagagg ccacgaacat gc 22
<210> 17
   <211> 23
   <212> DNA
   <213> Artificial Sequence
<220>
   <223> Primer
<400> 17
   cagcctactg gagatcagga tga 23
<210> 18
   <211> 22
   <212> DNA
   <213> Artificial Sequence
<220>
   <223> Primer
<400> 18
   ggagtccttg gatgagtctc ga 22
<210> 19
   <211> 24
   <212> DNA
   <213> Artificial Sequence
<220>
   <223> Primer
<400> 19
   gcgaactcac acaggcgaga aacc 24
<210> 20
   <211> 23
   <212> DNA
   <213> Artificial Sequence
<220>
   <223> Primer
<400> 20
   tcgcttcctc ttcctccgac aca 23
<210> 21
   <211> 19
   <212> DNA
   <213> Artificial Sequence
<220>
   <223> Primer
<400> 21
   cccattgccc ctgctcctg 19
<210> 22
   <211> 19
   <212> DNA
   <213> Artificial Sequence
<220>
   <223> Primer
<400> 22
   tgtgtccgcc ggtgtctgg 19

## Description

### FIELD OF INVENTION

This invention falls within the field of molecular biology, biotechnology and medicine. More particularly, it relates to gene therapy for the amelioration of certain markers of ageing in mammals, and the extension of lifespan.

### BACKGROUND OF THE INVENTION

A major goal in ageing research is to improve health during ageing. An increase in lifespan has little meaning if it is not linked to the preservation of a healthy condition or an amelioration of various age-related parameters (the so-called "health-span"). Often, the preservation or amelioration of health-span is accompanied by an increase of lifespan (Bluher, Kahn *et al.* 2003; Conboy, Conboy *et al.* 2005; Tomas-Loba, Flores *et al.* 2008).

Ageing is a multifactorial process that has been adjusted by nature to a wide spectrum of lifespans, even in closely related species, thus suggesting that ageing is a flexible trait susceptible to the influence of a number of molecular pathways (Brown-Borg et al., 1996. Nature 384, 33; Haigis & Guarente, 2006. Genes Dev. 20, 2913-2921; Kenyon, 2005. Cell 120, 449-460). One of such processes is the progressive attrition of telomeres that occurs associated with organismal ageing in humans (Harley et al., 1990. Nature 345, 458-460) and in other mammals, such as mice (Flores et al, 2008. Genes and Dev 22, 654-667). Telomeres are specialized structures at the ends of chromosomes, which have a role in protecting the chromosome ends from DNA repair and degrading activities (Blackburn, 2001. Cell 106, 661-673; de Lange, 2005. Genes Dev. 19, 2100-2110). Mammalian telomeres consist of TTAGGG repeats bound by a multi-protein complex known as shelterin (de Lange, 2005. Genes Dev. 19, 2100-2110). A minimum length of TTAGGG repeats and the integrity of the shelterin complex are necessary for telomere protection (Blackburn, 2001. Cell 106, 661-673; de Lange, 2005. Genes Dev. 19, 2100-2110). Telomerase is a cellular reverse transcriptase (TERT, telomerase reverse transcriptase; also known as TP2; TRT; EST2; TCS1; hEST2) capable of compensating telomere attrition through *de novo* addition of TTAGGG repeats onto the chromosome ends by using an associated RNA component as template *(Terc,* telomerase RNA component) (Greider and Blackburn, 1985. Cell 43, 405-413). Telomerase is expressed in most adult stem cell compartments, however, this is not sufficient to maintain telomere length as evidenced by the fact that telomere shortening occurs with age in most human and mouse tissues (Harley et al., 1990. Nature 345, 458-460; Blasco, 2007. Nat Chem Biol. 3, 640-649; Flores et al., 2008. Genes and Dev 22, 654-667).

Accumulation of short/damaged telomeres with increasing age is considered one of the main sources of ageing-associated DNA damage capable of causing loss of the regenerative capacity of tissues and systemic organismal ageing (Flores, Cayuela *et al.* 2005; Schoeftner, Blanco *et al.* 2009). This is supported both by the study of the telomerase-deficient mouse model as well as of human diseases characterized by mutations in telomerase components, which suffer from premature adult stem cell dysfunction and decreased longevity due to accelerated rates of telomere shortening even at the first generation (Blasco, Lee *et al.* 1997; Lee, Blasco *et al.* 1998; Herrera, Samper *et al.* 1999; Mitchell, Wood *et al.* 1999; Vulliamy, Marrone *et al..* 2001; Yamaguchi, Calado *et al.* 2005; Garcia-Cao, Garcia-Cao *et al.* 2006; Armanios, Chen *et al.* 2007; Tsakiri, Cronkhite *et al.* 2007). This evidence strongly suggests that telomerase activity and telomere length are rate-limiting for mammalian lifespan, and support a model in which short telomeres actively contribute to ageing by limiting tissue renewal. In this model, telomerase acts as a longevity gene in the context of the organism by preventing premature telomere attrition associated with age. Telomerase, indeed, confers indefinite proliferative potential to *in vitro* cultured cells by virtue of its ability to elongate chromosome ends, thus preventing critical telomere erosion associated with cell division and the subsequent activation of a persistent DNA damage response (Counter, Avilion *et al.* 1992; Bodnar, Ouellette *et al.* 1998; Counter, Hahn *et al.* 1998).

An important prediction of this model is that slowing the rate of telomere shortening should delay ageing. Thus, telomerase activation is envisioned as a potential strategy to rejuvenate tissues, as well as for the treatment of diseases characterized by premature telomere shortening.

However, telomere shortening is an important barrier for the uncontrolled proliferation of tumour cells (Feldser and Greider, 2007. Cancer Cell 11, 461-469; Blasco, 2005. Nat Rev Genet 6, 611-622), and increased telomerase expression is associated with a higher susceptibility to develop cancer both in mice and humans. Over-expression of the telomerase reverse transcriptase, TERT, is a common feature of human cancers, which seems to be associated with the telomerase ability to confer with unlimited proliferative potential. Not surprisingly, TERT over-expression has been demonstrated to increase cancer incidence in the context of classical mouse TERT transgenesis, where the transgenic mice are usually under the effects of the constitutive telomerase expression from early stages of development. Remarkably, over-expression of TERT in the context of mice engineered to be cancer-resistant *(e.g.* Sp53/Sp16/SARF/TgTERT mice) is sufficient to decrease telomere damage with age, delay ageing, and increase median longevity by 40% (Tomas-Loba, Flores *et al.* 2008).

However, risk of causing cancer has dissuaded researchers from envisioned gene therapy approaches as a valid strategy to delay ageing or to extend longevity.

In order to avoid the risk of tumours associated with telomerase over-expression, many studies relating to the possible use of telomerase in the amelioration of the marker associated with ageing have focused on the identification and development of pharmacological activators of telomerase activity. Telomerase activation is currently envisioned as a potential strategy to rejuvenate tissues, as well as for the treatment of diseases characterized by premature telomere shortening. However, no telomerase-activating drugs have been reported yet, only nutritional activators (Fauce, Jamieson *et al.* 2008). Their mechanism of action is still poorly understood and it is not clear if they have an influence in increasing cancer risk or, even, if they are capable of improving healthspan and/or lifespan.

In this context, the utility of telomerase or telomerase-related treatments in the improvement of the healthspan and the lifespan remains doubtful. However, it would be extremely valuable to find a way of ameliorating certain disorders that appear often in adult population, such as osteoporosis, glucose intolerance and neuromuscular degeneration associated with loss of neuromuscular coordination (which are well established indicators of ageing progression, are considered to be markers of the healthspan and seem to be connected to telomere shortening), in a simple and effective manner, without increasing the risk of spontaneous tumours. Any such solution would be of particular interest if, in addition of ameliorating those disorders, it had a positive influence in extending the expected lifespan of the treated individuals.

The present invention provides a solution to these problems.

### SUMMARY OF THE INVENTION

The invention is based on the surprising finding that gene therapy intended to increase the expression of telomerase reverse transcriptase, when administered to adult mammals, significantly extends both the average and maximum lifespan. Furthermore, this effect is achieved without significantly increasing the incidence of cancer (malignant neoplastic disease), as assessed by the number of spontaneous neoplasms evident among the population treated. The extension of lifespan is accompanied by a clear amelioration of some markers of healthspan that are conventionally considered markers of ageing, such as, osteoporosis, glucose intolerance with insulin insensitivity, loss of memory, and neuromuscular degeneration associated with loss of neuromuscular coordination.

The assays and results set forth in the Examples of the present application demonstrate the capacity of a telomerase-based gene therapy to improve healthy ageing and extend lifespan and healthspan longevity in an adult or aged mammalian organism. As such, this work constitutes a proof of principle for the feasibility of anti-ageing gene therapy. The anti-ageing effects provided by the invention have been observed by the inventors when telomerase is expressed from a non-integrative and post-mitotic tissue-targeting gene therapy vector administered to adult (1 year old) and aged (2 year old) mice, specifically from an adeno-associated virus-based expression vector. The beneficial effects lasted at least for 9 months in the case of mice treated at 1 year of age. Telomerase-treated mice, both at 1 year and at 2 years of age, have an increase in median lifespan of 24% and 13%, respectively. Survival curves were completed in the case of mice treated at 2 years of age and they also presented a significant increase (20%) in the maximal lifespan.

In summary, TERT increased expression has beneficial effects on tissues both through the canonical pathway (by preventing critical telomere loss and loss of cell viability), and through the non-canonical pathway (via Wnt/β-catenin stimulation of self-renewing capabilities of adult tissues). The present application thus provides proof of principle for the feasibility of anti-ageing interventions on adult/old mammals. Aged organisms accumulate telomere-derived DNA damage and it is demonstrated herein that it is possible to repair or delay the accumulation of this type of damage through telomerase gene therapy. This has direct consequences on the health of the aged organisms including an increase in the average and maximal lifespan.

Accordingly, the invention provides a nucleic acid vector comprising a coding sequence for telomerase reverse transcriptase (TERT) wherein
the treatment does not significantly increase the incidence of cancer in the subject,
the nucleic acid vector is a non-integrative and post-mitotic tissue targeting vector,
the adult is a human
and
the age-related disorder is selected from the group of osteoporosis, glucose intolerance, insulin resistant, loss of memory, and loss of neuromuscular coordination, or combinations thereof.

In the present invention, it is preferred that the TERT sequence used in the gene therapy vector is derived from the same species as the subject. For example, gene therapy in humans would be carried out using the human TERT sequence. Gene therapy in mice would be carried out using the mouse TERT sequence, as described in the examples. In one embodiment, the TERT is encoded by the nucleic acid sequence as set forth in SEQ ID NO:1, or SEQ ID NO: 3 (human TERT variants 1 and 2), or is an active fragment or functional equivalent of SEQ ID NO: 1 or SEQ ID NO: 3. The polypeptide sequence encoded by SEQ ID NO: 1 is set forth in SEQ ID NO: 2. The polypeptide encoded by SEQ ID NO: 3 is set forth in SEQ ID NO: 4.

As used herein, "functional equivalent" refers to a nucleic acid molecule that encodes a polypeptide that has TERT activity or a polypeptide that has TERT activity. The functional equivalent may display 50%, 60%, 70%, 80%, 90%, 95%, 98%, 99%, 100% or more activity compared to TERT encoded by SEQ ID NO: 1 or SEQ ID NO: 3. Functional equivalents may be artificial or naturally-occurring. For example, naturally-occurring variants of the TERT sequence in a population fall within the scope of functional equivalent. TERT sequences derived from other species also fall within, the scope of the term "functional equivalent", in particular the murine TERT sequence given in SEQ ID NO: 5. The functional equivalent may be a nucleic acid with a nucleotide sequence having at least 75%, 80%, 85%, 90%, 95%, 96%, 97%, 98%, 99%, 99.5%, 99.9% identity to SEQ ID NO: 1 or SEQ ID NO: 3. The functional equivalent may be a polypeptide with an amino acid sequence having at least 75%, 80%, 85%, 90%, 95%, 96%, 97%, 98%, 99%, 99.5%, 99.9% identity to SEQ ID NO: 2 or SEQ ID NO: 4. In the case of functional equivalents, sequence identity should be calculated along the entire length of the nucleic acid. Functional equivalents may contain one or more, e.g. 2, 3, 4, 5, 10, 15, 20, 30 or more, nucleotide insertions, deletions and/or substitutions when compared to SEQ ID NO: 1 or SEQ ID NO: 3.

The term "functional equivalent" also encompasses nucleic acid sequences that encode a TERT polypeptide with at least 75%, 80%, 85%, 90%, 95%, 96%, 97%, 98%, 99%, 99.5%, 99.9% sequence identity to the sequence as set forth in SEQ ID NO:2 or SEQ ID NO: 4, but that show little homology to the nucleic acid sequence given in SEQ ID NO: 1 or SEQ ID NO: 3 because of the degeneracy of the genetic code.

As used herein, the term "active fragment" refers to a nucleic acid molecule that encodes a polypeptide that has TERT activity or polypeptide that has TERT activity, but which is a fragment of the nucleic acid as set forth in SEQ ID NO: 1 or SEQ ID NO: 3 or the amino acid sequence as set forth in SEQ ID NO: 2 or SEQ ID NO: 4. An active fragment may be of any size provided that TERT activity is retained. A fragment will have at least 75%, 80%, 85%, 90%, 95%, 96%, 97%, 98%, 99%, 99.5%, 100% identity to SEQ ID NO: 1-4 along the length of the alignment between the shorter fragment and SEQ ID NO: 1-4.

Fusion proteins including these fragments may be utilised. For example, an additional 5, 10, 20, 30, 40, 50 or even 100 amino acid residues from the polypeptide sequence, or from a homologous sequence, may be included at either or both the C terminal and/or N terminus without prejudicing the ability of the polypeptide fragment to fold correctly and exhibit biological activity.

Sequence identity may be calculated by any one of the various methods in the art, including for example BLAST (Altschul SF, Gish W, Miller W, Myers EW, Lipman DJ (1990). "Basic local alignment search tool". J Mol Biol 215 (3): 403-410) and FASTA (Lipman, DJ; Pearson, WR (1985). "Rapid and sensitive protein similarity searches". Science 227 (4693): 1435-41; http://fasta.bioch.virginia.edu/fasta_www2/fasta_list2.shtml) and variations on these alignment programs.

In one aspect of the desclosure the method of applying the treatment in which the vector comprising a coding sequence for telomerase reverse transcriptase is for use is a gene therapy method. Gene therapy methods and vectors are well known in the art and generally comprise delivering a nucleic acid encoding a therapeutically active protein to a subject. The nucleic acid may be delivered in a number of ways including delivering naked DNA such as plasmid or mini-circles, the use of liposomes or cationic polymers or other engineered nano-particles containing the nucleic acid, or viral vectors that encapsidate the nucleic acid.

Gene thereapy may be achieved using stable transformation of organisms with an inducible expression system. This aspect of the disclosure does not extend to human subjects. Expression of TERT can be induced at a later date following transformation, for example, once the subject is an adult or an aged adult, or begins to show signs of age-related disorders. Suitable inducible expression systems are known in the art and include the CRE-LOX recombinase based system which is suitable for use in mice and tetracycline-regulated which can be used in the treatment of human subjects.

As discussed above, over-expression of TERT in transgenic animals is known to lead to an increase in the incidence of cancer in the transgenic animals. Without wishing to be bound by theory, the inventors believe that this is due at least in part by the over-expression of TERT at all stages of development. The present invention is limited to the expression of TERT in an adult which is a human. By limiting the present invention to be for use in the treatment of a human adult, and to the use of vectors targeting post-mitotic cells within the subjects, the invention avoids the problem of increased incidence of cancer that is known in the art.

The results disclosed in the present application show that viral gene therapy vectors provide an efficient way to deliver TERT expression systemically to many tissues. Thus, in a preferred aspect of the present disclosure, the gene therapy vector is a viral vector.

Viral gene therapy vectors are well known in the art. Vectors for use in the present invention include non-integrative vectors such as those based on retroviruses, adenoviruses (AdV), adeno-associated viruses (AAV), lentiviruses, pox viruses, alphaviruses, and herpes viruses. The inventors of the present invention have demonstrated the principle of the invention using an AAV vector.

Using non-integrative viral vectors, such as AAV, seems to be particularly advantageous. In one aspect, this is because non-integrative vectors do not cause any permanent genetic modification. Second, the vectors target to adult tissues, avoiding having the subjects under the effect of constitutive telomerase expression from early stages of development. Additionally, non-integrative vectors effectively incorporate a safety mechanism to avoid over-proliferation of TERT expressing cells. Cells will lose the vector (and, as a consequence, the telomerase expression) if they start proliferating quickly.

Particular examples of suitable non-integrative vectors include those based on adenoviruses (AdV) in particular gutless adenoviruses, adeno-associated viruses (AAV), integrase deficient lentiviruses , pox viruses, alphaviruses, and herpes viruses. Preferably, the non-integrative vector used in the invention is an adeno-associated virus-based non-integrative vector, similar to natural adeno-associated virus particles. AAV preferentially targets post-mitotic tissues, which are considered more resistant to cancer than the highly proliferative ones. Examples of adeno-associated virus-based non integrative vectors include vectors based on any AAV serotype, i.e. AAV1, AAV2, AAV3, AAV4, AAV5, AAV6, AAV7, AAV8, AAV9, AAV10, AAV11 and pseudotyped AAV. Tissue specificity is determined by the capsid serotype. Pseudotyping of AAV vectors and capsid engineering to alter their tropism range will likely be important to their use in therapy.

Vectors derived from adeno-associated viruses (AAVs) have emerged as one of the vectors of choice for many gene transfer applications because of their many desirable properties, including capability to transduce a broad range of tissues at high efficiency, poor immunogenicity and an excellent safety profile (Merten, Geny-Fiamma *et al.* 2005; Buning, Perabo *et al.* 2008), toxicity being absent in many preclinical models (Niemeyer, Herzog et al Blood 2009; Mas, Montane et al Diabetes 2006; Jiang, Lillicrap et al blood 2006; Ghosh, Yue et al Molecular therapy 2007; Tafuro, Ayuso et al cardiovascular research 2009). AAV vectors transduce post-mitotic cells and can sustain long-term gene expression (up to several years) both in small and large animal models of disease (Niemeyer, Herzog et al Blood 2009; Mas, Montane et al Diabetes 2006; Jiang, Lillicrap et al blood 2006; Ghosh, Yue et al Molecular therapy 2007; Tafuro, Ayuso et al cardiovascular research 2009). Safety and efficacy of AAV gene transfer has been extensively studied in humans with encouraging results in the liver, muscle, CNS, and retina (Manno et al Nat medicine 2006, Stroes et al ATVB 2008, Kaplitt, Feigin, Lancet 2009; Maguire, Simonelli et al NEJM 2008; Bainbridge et al. NEJM 2008).

AAV2 is the best characterized serotype for gene transfer studies both in humans and experimental models. AAV2 presents natural tropism towards skeletal muscles, neurons, vascular smooth muscle cells and hepatocytes. AAV2 is therefore a good choice of vector to target these tissues, in particular for use according to the invention in the treatment of an age-related disorder associated with one of these tissues. For example, treatment of neuromuscular degeneration may be targeted to skeletal muscle and/or neurons in this way.

Newly isolated serotypes, such as AAV7, AAV8, and AAV9 have been successfully adopted in preclinical studies (Gao, Alvira et al PNAS 2002), and are likely to enter Phase I clinical trials in the near future. Although limited immunologic responses have been detected in human subjects treated with AAV2 or AAV1 against the AAV capsid (Manno et al Nat Med 2006; Mingozzi et al. Nat Med 2007; Brantly et al PNAS 2009; Mingozzi et al blood 2009), long term expression of the therapeutic gene is possible depending on the target tissue and the route of administration (Brantly et al PNAS 2009; Simonelli et al mol therapy 2010). In addition, the use of non-human serotypes, like AAV8 and AAV9, might be useful to overcome these immunological responses in subjects, and clinical trials have just commenced (ClinicalTrials.gov Identifier: NCT00979238). Altogether, these encouraging data suggest that AAV vectors are useful tools to treat human diseases with a high safety and efficient profile.

The choice of adeno-associated viruses of wide tropism, such as those derived from serotype 9 adeno-associated virus (AAV9) is particularly advantageous when treating age-related disorders in general. AAV9 viruses have shown efficient transduction in a broad range of tissues, with high tropism for liver, heart and skeletal muscle (Inagaki et al Molecular Therapy 2006) and thus the beneficial effects of gene therapy can be achieved in more tissues. In addition, AAV9 vectors have the unique ability to cross the blood-brain-barrier and target the brain upon intravenous injection in adult mice and cats (Foust et al Nature biotechnology 2009; Duque *et al* Molecular therapy *et al* 2009).

Furthermore, it has been observed that tissue transduction with AAV9 vectors does not have any negative impact on the longevity of mice, as indicated by a normal survival of AAV9-eGFP treated mice. Indeed, the results described here in mice show that gene therapy interventions with expression of TERT at 1 year or 2 years of age have clear beneficial effects on many different aspects of mouse health, including delayed osteoporosis, improved epithelial barrier fitness, improved glucose tolerance, improved memory function, and improved neuromuscular coordination.

Notably, these signs of delayed ageing are accompanied by a significant increase in longevity of approximately 24% and 13% in the 1 year old and 2 year old groups, respectively. Maximum longevity is also increased by 5 months in the 2 year old group treated with AAV9-mTERT compared to AAV9-eGFP treated controls, which represents a 20% increase in maximum longevity (182 weeks vs 155 weeks, respectively).

Importantly, AAV9-mTERT treated mice did not develop increased incidence of cancer, illustrating the safety of this type of strategy. This is likely to be related to the fact that AAV vectors are non-integrative, and therefore mTERT over-expression will be lost in highly proliferating cells. In addition, AAV preferentially targets post-mitotic tissues, which are considered more resistant to cancer than the highly proliferative ones.

Thus, AAV9-derived vectors are the preferred vectors for the embodiments of the invention, because they allow a broad expression of TERT in adult mice and other mammals such as humans, making it possible to apply the beneficial effects of telomerase expression on healthspan and longevity to a wide variety of tissues and species.

Accordingly, a vector for use according to the invention may involve a system in which the capsid (which is the part of the virus which determines the virus tropism) of the adeno-associated virus-based vector is made of capsid proteins of the serotype 9 adeno-associated virus (AAV9). In vectors for use according to preferred embodiments of the invention, the polynucleotide sequence packed in the capsid is flanked by internal terminal repeats (ITRs) of an adeno-associated virus, preferably of serotype 2 which has been extensively characterised in the art, and presents a coding sequence located between the ITRs. As set out above, the nucleic acid codes for a functional TERT polypeptide. In one particularly preferred embodiment, the regulatory sequence operatively linked to the TERT coding sequence is the cytomegalovirus promoter (CMV), although other suitable regulatory sequences will be known to those of skill in the art.

When treating specific age related disorders, it is advantageous to target the treatment to the effected tissues. The choice of AAV serotype for the capsid protein of the gene therapy vector may be thus based on the desired site of gene therapy. If the target tissue is skeletal muscle, for example, in treating loss of neuromuscular coordination, AAV1-and AAV6-based viral vectors can be used. Both of these serotypes are more efficient at transfecting muscle than other AAV serotypes. AAV3 is useful for transfecting haematopoietic cells. A thorough review of AAV-based vectors for gene therapy can be found in Shi et al, (2008) "AAV-based targeting gene therapy" Am. J. Immunol. 4:51-65. Other viral vectors with the required properties can be used in the present invention. Heilbronn & Weger (2010) Handb Exp Pharmacol. 197:143-70 provides a review of viral vectors that are useful in gene therapy.

The present disclosure describes a vector comprising a coding sequence for telomerase reverse transcriptase (TERT) suitable for use in gene therapy. Gene therapy vectors include any kind of particle that comprises a polynucleotide fragment encoding the telomerase reverse transcriptase (TERT) protein, operably linked to a regulatory element such as a promoter, which allows the expression of a functional TERT protein demonstrating telomerase reverse transcriptase activity in the targeted cells. Preferably, TERT is encoded by the nucleic acid sequence as set forth in SEQ ID NO: 1 or SEQ ID NO: 3, or is an active fragment or functional equivalent of TERT.

The term gene therapy vector in general includes within its scope naked DNA molecules such as plasmids or mini-circles, i.e. circular DNA molecules which do not contain bacterial DNA sequences, provided that the TERT coding sequence and its linked regulatory element are inserted in the plasmid, as well as to more complicated systems, such as particles with the structure of virions (viral particles), comprising at least a capsid and at least a polynucleotide sequence, with a size that allows the polynucleotide sequence to be packed within the capsid in a manner similar to that of the native genome of the virus of origin of the capsid. The polynucleotide sequence must include a region where the TERT coding sequence and its linked regulatory element are inserted such that the telomerase reverse transcriptase protein can be expressed from that polynucleotide sequence once the viral particle has infected a cell.

A gene therapy vector for use according to the invention is a non-integrative vector as defined in claim 1, such as an adeno-associated virus-based non-integrative vector. For the purposes of then invention, the choice of non-integrative vectors seems to be particularly advantageous, because they do not cause any permanent genetic modification. Also, as stated before, such vectors incorporate a safety mechanism to avoid over-proliferation of TERT expressing cells, that will lose the vector if the cells start proliferating quickly.

Adeno-associated virus-based vectors derived from a serotype 9 adeno-associated virus (AAV9) are preferred because the beneficial effects can be achieved in more tissues (see above). In one particularly preferred embodiment, the regulatory sequence operatively linked to the TERT coding sequence is the cytomegalovirus promoter (CMV).

The nucleic acid sequence encoding TERT is operably linked to a regulatory sequence that drives the expression of the coding sequence. As used herein, the term "regulatory element" means a nucleic acid sequence that serves as a promoter, i.e., regulates expression of a nucleic acid sequence operably linked to the promoter. Such "regulatory elements" or "promoters" can control the expression of linked nucleic acid sequences either constitutively or inducible.

The regulatory sequence may be a constitutive promoter. An example of a regulatory sequence which is a constitutive promoter is the cytomegalovirus (CMV) promoter.

The TERT-based gene therapy of the present invention significantly improves several molecular markers of ageing. In the Examples included herein, as expected from the canonical function of TERT as the catalytic component of telomerase, mice treated with AAV9-mTERT vectors showed a significant telomere elongation in a variety of tissues, which was concomitant with a significant decrease in the abundance of short telomeres. It is worth mentioning here that the presence of short telomeres, rather than a decreased average telomere length, forms the ultimate cause of chromosomal instability (Hemann, Strong *et al.* 2001; Samper, Flores *et al.* 2001). On the other hand, TERT has been recently shown to have telomere-independent roles as an activator of some genes of the Wnt pathway through interaction with BRG1 (Millar 2009; Park, Venteicher *et al.* 2009), which in turn may mediate some of the previously known effects of TERT improving stem cell mobilization (Flores, Cayuela *et al.* 2005; Reya and Clevers 2005; Sarin, Cheung *et al.* 2005). Notably, the results of the assays of the Examples of the present application show that tissues with increased TERT expression also showed increased expression of active β-catenin, as well as of its target gene cyclin D1. No consistent increase in other Wnt target genes previously related to TERT, such as Axin2 and CD44, was observed, although its expression greatly varied depending on the tissue type. Notably, increased cyclinD1 was also paralleled by generally lower levels of p16 expression in some mouse tissues, a further sign of delayed ageing and extended renewal and proliferative capacities associated with TERT expression.

The present disclosure is effective to extend both lifespan and healthspan.

As used herein, the term "lifespan" encompasses both the maximum lifespan (i.e. the maximum age that any member of a particular species has attained) and the average lifespan. The average lifespan may be the mean lifespan, or the median lifespan.

Lifespan may be extended by 5%, 10%, 15%, 20% or more, with reference to the expected lifespan for that species, including humans. Extended lifespan may be an increase in the maximum lifespan possible for any particular species of subject. Extended lifespan may be an increase in the average lifespan of an individual of that species who reaches adulthood. Thus, extended lifespan may be a 5%, 10%, 15%, 20% or more increase in maximum lifespan and/or a 5%, 10%, 15%, 20% or more increase in average lifespan.

For the human, the maximum lifespan is thought to be about 120 years and the average lifespan for an individual reaching adulthood in a developed nation is about 70-80 years depending on a number of factors including sex of the individual, nutrition and locality. In terms of years, it is expected that lifespan may be extended by 1, 2, 5, 10, 15, or even 20 or more years.

An increase in lifespan has little meaning if it is not linked to the preservation of a healthy condition or an amelioration of various age-related parameters. The invention also extends healthspan.

As used herein, the "healthspan" is the period of time for which an individual is generally healthy and free of chronic illness. In particular, an individual will be substantially free from age-related disorders during his or her healthspan. By extending the healthspan, the present invention leads to a rise in the average age of onset of age-related disorders as compared to an untreated population.

The present disclosure extends the period of time for which an individual is generally healthy and free of chronic illness and/or ameliorates disorders that appear often in aged and ageing adult population, including reduced epithelial barrier fitness, osteoporosis, glucose intolerance and neuromuscular degeneration associated with loss of neuromuscular coordination. These are well established indicators of ageing progression, and are considered to be markers of the healthspan.

Accordingly, the disclosure has beneficial effects in at least one of the following group: reducing the incidence of cancer, on delaying and/or ameliorating osteoporosis, improving epithelial barrier fitness, improving glucose tolerance, improving memory function, and improving neuromuscular coordination. The amelioration of age-related disorders provided by the disclosure can be as a result of reduction of symptoms in an affected subject or a reduction of incidence of the disease or disorder in a population as compared to an untreated population.

One important feature of the invention is that the gene therapy associated to the invention is achieved without significantly increasing the incidence of cancer, as assessed by the number of spontaneous tumours that are evident among the population treated. In the invention, the vector comprising a coding sequence for telomerase reverse transcriptase is for use in the treatment of at least one of the mentioned disorders *without* the concomitant use of a cancer suppressor.

This is unexpected for the person of skill in the art, having knowledge of results previously obtained with transgenic mice over-expressing TERT. These mice show a significant increase in spontaneous tumours, a principal reason why activation or over-expression of TERT has not been pursued in the art for the treatment of ageing. The only exception to this tumour incidence has been with telomerase over-expression in mice genetically-engineered to be cancer resistant (Tomas-Loba, Flores *et al.* 2008). Indeed, a TERT-based gene therapy strategy to extend longevity is unprecedented in the context of ageing studies. Although gene therapy approaches, in general, are envisioned as an effective way to deliver genes into adult tissues in order to correct genetic defects or diseases, gene therapy has never to our knowledge been envisioned as a valid strategy to delay ageing or extend longevity.

Although the inventors do not wish to be bound by any theory, the lack of increased incidence of cancer in test animals described herein, compared to control littermates and to conventional TERT transgenic mice, may be due to the fact that the gene therapy vector was not administered to the individuals during embryo development, but was administered comparatively late in life. The difference from the prior art might arise from the fact that, in earlier studies with transgenic mice, increased TERT expression is forced from the stage of early embryo development through germline modifications, which may favour the expansion of cancerous cells and the development of cancer later in life. Consistently with that hypothesis, the authors of the invention set to address whether increased TERT expression later in life (in adult and old mice) has rejuvenating effects without increasing cancer risk. This scenario would be analogous to that of the approach on which more efforts have been focused lately, that of using pharmacological activators of telomerase for the treatment of age-related diseases or for increasing the health span of individuals. Telomerase-based gene therapy, therefore, is an alternative to the use of telomerase activators. That approach is currently being hindered by difficulties in finding suitable pharmacological activators of the telomerase.

Accordingly, it is a feature of the invention that only adults are to be treated with the nucleic acid vector according to the present invention. Constitutive treatment throughout subject life is explicitly excluded from the invention. Consistent with this theory, the invention uses a viral vector, as explained in detail above.

The expression of TERT following gene therapy using the vectors according to the invention persists for a time of several months to several years. In mice, TERT expression was detectable after 5 months. In monkey, gene expression following gene therapy with an AAV-based vector has been detected up to 6 years after treatment and up to 8 years in dogs (Rivera et al Blood 2005, and Niemeyer et al blood 2009). Frequent repetition of treatment with the vectors according to the invention is therefore not necessary. The subject may be treated once. The subject may be treated initially, and then treated again once TERT expression levels decrease by about 50% of those attained immediately following treatment. Treatment may be repeated with the same or alternative vector to maintain the reduction in age-related disorders if necessary, for example annually, or once every 5 years or once a decade. When administering a second or subsequent dose, it may be necessary to use a different gene therapy vector, for example when using an AAV-based vector the second and subsequent administrations may be a vector with a capsid derived from a different serotype than that used for the first administration. It is possible that a subject may develop neutralising antibodies to the first gene therapy vector, making it ineffective if administered a second or subsequent time (Amado et al (2010) Science Translational Medicine 2(21):21ra16).

Unexpectedly, the present disclosure when used in the treatment of age-related disorders, leads to a reduction in the incidence of cancer. Reduction in the rate of incidence of cancer can be measured by comparing the rate of a particular cancer in a treated population to the rate of the same type of cancer in an untreated control population.

The gene therapy method associated to the disclosure has the effect of treating and/or preventing various age-related conditions and diseases, as assessed by particular markers and disorders of ageing. The disclosure relates to a gene therapy vector, as described in any one of the embodiments set out above, for use in the treatment or prevention in a human adult subject of at least a disorder or marker of ageing that is selected from the group of reduced epithelial barrier fitness, osteoporosis, glucose intolerance, insulin resistance, loss of memory, loss of neuromuscular coordination, , or combinations thereof. The gene therapy ameliorates at least one marker of ageing, selected for example, from the group of reduced epithelial barrier fitness, osteoporosis, arthrosis, glucose intolerance, insulin resistance, cardiovascular disease, reduced heart and circulatory function, reduced lung function, loss of memory, loss of neuromuscular coordination or decrease of longevity or combinations thereof.

Osteoporosis results in decreased bone density and an increase in the frequency of fractures. Thus, the invention can be used for the treatment or prevention of osteoporosis by reducing either of these measures. The onset of osteoporosis may be delayed. The age of onset of osteoporosis may be increased by 10%, 20%, 30%, 40%, 50%, 60%, 70%, 80% or 90% or more, and may be 1, 2, 3, 5, 7, 10, 15, 20 years or more. If osteoporosis does in due course occur, the invention reduces the rate of decrease in bone density and/or frequency of fractures.

Bone density can be measured as described herein using the DEXA scan device, or can be measured by any other suitable means known in the art. By "reduces the rate of decrease in bone density" it is meant that the rate of loss of bone density in a treated subject is less than the rate of decrease in an equivalent untreated subject, or less than the average decrease in an equivalent population of the same age range. The rate of decrease in bone density in a treated subject may be 90%, 80%, 70%, 60%, 50%, 40%, 30%, 20% or 10% that of an untreated subject. Typically, bone density in a health adult ranges from 1200mg/cm² to 950 mg/cm² in a healthy adult human depending on gender and ethnicity. Depending on the start point, bone density decreases proportionately, for example to the range of about between 1100 and 850 mg/cm² in a subject may be considered to be aged.

Arthrosis, also known as osteoarthritis, described a group of mechanical abnormalities involving degradation of joints, including articular cartilage and subchondral bone. Symptoms may include joint pain, tenderness, stiffness, locking, and also effusion. The disclosure can be used in the treatment or prevention of arthrosis by reducing any of these measures. In one embodiment, the disclosure delays the onset of arthrosis. In one embodiment, the age of onset of arthrosis is increased by 10%, 20%, 30%, 40%, 50%, 60%, 70%, 80% or 90% or more, and may be 1, 2, 3, 5, 7, 10, 15, 20 years or more.

Arthrosis can be determined by X-ray, in particular by the observation of the following indicia: joint space narrowing, subchondral sclerosis (increased bony formation around the joint), subchondral cyst formation, and osteophytes, or can be measured by any other suitable means known in the art.

Glucose intolerance is also referred to in the art as impaired glucose tolerance and is a pre-diabetic state of dysglycemia that is associated with insulin resistance and increased risk of cardiovascular disease. Glucose tolerance can be measured using the glucose tolerance test as described herein. In humans, the glucose tolerance test as describe in Definition, diagnosis and classification of *diabetes mellitus* and its complications: World Health Organization and International Diabetes Federation (1999). By "improving glucose intolerance" or "ameliorating glucose intolerance" in the context of the invention it is meant that the incidence of glucose intolerance may be decreased in a treated population when compared to a control untreated population of the same age range. In one embodiment, the age of onset of glucose intolerance is increased by 10%, 20%, 30%, 40%, 50%, 60%, 70%, 80% or 90% or more, and may be 1, 2, 3, 5, 7, 10, 15, 20 years or more. In a particular embodiment, the occurrence of glucose intolerance in a treated population is 10%, 20%, 30%, 40%, 50%, 60%, 70%, 80% or 90% that of an untreated population. Alternatively, "improving glucose intolerance" or "ameliorating glucose intolerance" can refer to reduced glucose intolerance in an individual treated subject compared to an untreated subject. In this embodiment, the 2 hour OGTT glucose level of an individual treated in accordance with the present invention should be ≤7.8 mmol/l (140 mg/dl).

Insulin resistance differs from glucose intolerance. Insulin resistance is measured using the "hyperinsulinemic euglycemic clamp", which measures the amount of glucose necessary to compensate for an increased insulin level without causing hypoglycaemia as decribed in DeFronzo R, Tobin J, Andres R (1979). "Glucose clamp technique: a method for quantifying insulin secretion and resistance". Am J Physiol 237 (3): E214-23. The rate of glucose infusion during the last 30 minutes of the test determines insulin sensitivity. If high levels (7.5 mg/min or higher) are required, the patient is insulin-sensitive. Very low levels (4.0 mg/min or lower) indicate that the body is resistant to insulin action. Levels between 4.0 and 7.5 mg/min indicate impaired glucose tolerance.

By "improving insulin resistance" or "ameliorating insulin resistance" in the context of the invention it is meant that the incidence of insulin resistance may be decreased in a treated population when compared to a control untreated population of the same age range. In one embodiment, the age of onset of insulin resistance is increased by 10%, 20%, 30%, 40%, 50%, 60%, 70%, 80% or 90% or more, and may be 1, 2, 3, 5, 7, 10, 15, 20 years or more. In a particular embodiment, the occurrence of insulin resistance in a treated population is 10%, 20%, 30%, 40%, 50%, 60%, 70%, 80% or 90% that of an untreated population. Alternatively, "improving insulin resistance" or "ameliorating insulin resistance" can refer to reduced insulin resistance in an individual treated subject compared to an untreated subject. In this embodiment, the rate of glucose infusion during the last 30 minutes of the test of an individual treated in accordance with the present invention should be ≥7.5 mg/min.

Improving memory function and ameliorating loss of memory can be assessed using a number of tests known in the art including The Mini Mental State Examination (MMSE) (Folstein MF, Folstein SE, McHugh PR; "Mini-mental state". A practical method for grading the cognitive state of subjects for the clinician. J Psychiatr Res. 1975 Nov;12(3):189-98), the Six Item Cognitive Impairment Test (6CIT) (Brooke P, Bullock R; Validation of a 6 item cognitive impairment test with a view to primary care usage. Int J Geriatr Psychiatry. 1999 Nov;4(11):936-40) or the Abbreviated Mental Test (AMT) (Hodkinson HM; Evaluation of a mental test score for assessment of mental impairment in the elderly. Age Ageing. 1972 Nov;1(4):233-8.) Improved memory function and/or ameliorated memory loss will result in an improvement in the score in any of the test given above. In the context of the invention, the improvement in memory function and/or amelioration of memory loss can be seen in an improvement of the average score in a memory test in a treated population when compared to an untreated control population. In a particular embodiment, the average memory test score in a treated population is 10%, 20%, 30%, 40%, 50%, 60%, 70%, 80% or 90% greater than that of an untreated population. Improvement in memory function and/or amelioration of memory loss can also be seen as an increase in the average age at which detectable memory loss first occurs. In one embodiment, the age of onset of memory loss, as assessed by any one of the above tests, is increased by 10%, 20%, 30%, 40%, 50%, 60%, 70%, 80% or 90% or more, and may be 1, 2, 3, 5, 7, 10, 15, 20 years or more.

Improvement in neuromuscular coordination can be assessed by one or more of the tests described in Dhesi JK et al. (Age Ageing. 2004;33(6):589-95). In the context of the invention, the improvement in neuromuscular coordination can be seen in an improvement of the average score in a neuromuscular coordination test in a treated population when compared to an untreated control population. The improvement may be 10%, 20%, 30%, 40%, 50%, 60%, 70%, 80% or 90% greater than that of the equivalent score in an untreated population of the same age.

Epithelial barrier fitness decreases with age. Decreased epithelial barrier fitness leads to an increase in the risk of skin injury, infection, and an increase in water loss and consequent dehydration. Epithelial barrier fitness can be analyzed by assessing morphological criteria of aging in epithelial tissues such as the skin and small intestine. The thinning of the subcutaneous adipose layer is a known aging-associated morphological feature, which, in turn, is associated with increased risk of skin injuries and infections. Further methods for measuring epithelial barrier fitness are described in Tomas-Loba et al (2008) Cell 135:609-622. The improvement may be 10%, 20%, 30%, 40%, 50%, 60%, 70%, 80% or 90% greater than that of the equivalent score in an untreated population of the same age. For example, the thickness of the epidermal layer in a treated individual will be between 40 and 60 µm, more preferably 50 to 60 µm.

Other markers of ageing include cardiovascular disease, heart and circulatory function and lung function as described in Woolf *et al.* Further markers of ageing are described in Balin AK et al "Practical handbook of human biologic age determination" CRC-press Boca Raton (1994).

Lung function can be expressed as forced vital capacity (FEV in 1 second). A healthy human adult has a vital capacity between 3 and 5 litres. After the age of 20 the vital capacity decreases approximately 250cm³ per ten years. The present invention is expected to give rise to improved lung function in aged subjects, and/or decreased decline in lung function. Preferably, the FEV of a treated aged subject is between 3 and 5 litres.

Heart and circulatory function can be measured using VO₂ max and/or blood pressure. An untrained healthy human adult typically has a VO₂ max in the range of 35 to 45 ml/kg/min. VO₂ max generally decreases with age. A healthy human adult typically has a systolic blood pressure in the range of 90 - 120 mmHg and a diastolic blood pressure in the range of 60-80 mmHg. Blood pressure generally increases with age. The present invention is expected to give rise to improved heart and circulatory function in aged subjects. Preferably, the VO₂ max of a treated aged subject is at least 30 ml/kg/min and more preferably is between 35 to 45 ml/kg/min and the systolic blood pressure of a treated aged adult is less than 120 mmHg and preferably between 90 - 120 mmHg and a diastolic blood pressure of a treated aged adult is less than 80mm Hg and is preferably between 60-80 mmHg.

The present invention is directed to gene therapy vectors for use in treating age-related disorders in a subject as defined in the claims. The subject is an adult human.

According to the present disclosure, an "adult" should be a fully developed individual who has attained reproductive ability, is fertile, or who evidences secondary sex characteristics. As used herein, the term adult when applied to humans for example, describes early adulthood commencing at around 20 years of age and extending to 39; middle adulthood (40 to 59) and late adulthood (60+). As a comparison, a one year old mouse can be considered to be approximately equivalent in age to a 45 year old human. A 2 year old mouse can be considered to be approximately equivalent to an 80 year old human.

An adult treated according to the invention may be aged. The term "aged" is applied to an individual who is older than the period of life during which the individuals of its species are generally healthy and free of chronic illness. Woolf et al in "Biologicaal Age Markers as Predictos for Health and Disease" (4th Amlasian Conference and Exhibition on Anti-Aging and Aesthetic Medicine) state that organ vitality reaches a peak between the ages of 15 and 25 for human females and 10 and 28 for human males. For the purposes of the present invention, "aged" is intended to describe human individuals of at least 25 years of age, but more preferably 45 years of age, 50 years of age, at least 60, at least 65, at least 70, 75, 80, 85, 90, 95 years or more.

The point at which a subject is considered to be aged can also be related to mean telomere length. For example, it is known in humans that telomere length decrease with age (Slagboom et al (1994) Am. J. Hum. Genet. 55:876-882). Mean telomere length in infancy and childhood is about 8kb or longer. This has decreased to about 7kb by the age of 40 and to about 6kb by the age of eighty. Thus a human subject can be considered to be aged when mean telomere length has decreased to about 7 kb.

Another measure of aging is the proportion of short telomeres compared to long telomeres. This can be measured as described herein in the examples. Telomere length can also be measured as described in Slagboom *et al* or Canela et al. (2007, PNAS 104:5300-5305). The proportion of short telomeres can be the fraction of telomeres presenting an intensity below 50% of the mean intensity or the fraction of telomeres below a certain length, e.g. 8kb, 7kb, 6kb, 5kb etc. A subject can be considered to be aged when 10%, 20%, 30%, 40%, 50%, 60% 70% or more of the telomeres in a sample derived from that subject are short, e.g. 7kb.

In the present disclosure, treatment of a subject will generally be immediately before the age when the subject is generally healthy and free of chronic illness, i.e. immediately before the subject is considered to be aged. In a human, treatment could therefore be started at 40, 45, 50, 60 years of age or older.

However, treatment can be administered at other times as well. When a subject is considered to be at particular risk of developing a specific age-related disorder, treatment may be performed before the onset of the age related disorder. For example, if a human subject is considered to be at high risk of developing heart disease at about the age of 40, treatment can be started at the age of 30, 35, 36, 37, 38, or 39. The same is true for other age-related disorders such as osteoporosis, glucose intolerance, loss of memory, loss of neuromuscular coordination.

In the present invention, it is preferred that the TERT sequence used in the gene therapy vector is derived from the same species as the subject. For example, gene therapy in humans would be carried out using the human TERT sequence. Gene therapy in mice would be carried out using the mouse TERT sequence, as described in the examples.

The present disclosure provides information for preparing a pharmaceutical composition comprising an effective amount of any one of the gene therapy vectors which are for use in the treatment or prevention of an age-related disorder according to the invention described above.

A "pharmaceutical composition" is intended to include the combination of an active agent with a carrier, inert or active, making the composition suitable for diagnostic or therapeutic *use in vitro, in vivo* or *ex vivo.*

"Composition" is intended to mean a combination of active agent and another compound or composition, inert (for example. a detectable agent or label) or active.

An "effective amount" is an amount sufficient to effect beneficial or desired results. An effective amount can be administered in one or more administrations, applications or dosages.

They will usually include components in addition to the active component (such as the gene therapy vector) *e.g.* they typically include one or more pharmaceutical carrier(s) and/or excipient(s). A thorough discussion of such components is available in Gennaro (2000) Remington: The Science and Practice of Pharmacy. 20th edition, ISBN: 0683306472.

Compositions will generally be administered to a subject in aqueous form. Prior to administration, however, the composition may have been in a non-aqueous form. For instance, although some viral vectors are manufactured in aqueous form, then filled and distributed and administered also in aqueous form, other viral vectors are lyophilised during manufacture and are reconstituted into an aqueous form at the time of use. Thus a composition suitable for the invention may be dried, such as a lyophilised formulation.

The composition may include preservatives such as thiomersal or 2-phenoxyethanol. It is preferred, however, that the composition should be substantially free from (*i.e.* less than 5µg/ml) mercurial material e.g. thiomersal-free.

To control tonicity, it is preferred to include a physiological salt, such as a sodium salt. Sodium chloride (NaCl) is preferred, which may be present at between 1 and 20 mg/ml e.g. about 10±2mg/ml NaCl. Other salts that may be present include potassium chloride, potassium dihydrogen phosphate, disodium phosphate dehydrate, magnesium chloride, calcium chloride, *etc.*

Compositions will generally have an osmolality of between 200 mOsm/kg and 400 mOsm/kg, preferably between 240-360 mOsm/kg, and will more preferably fall within the range of 290-310 mOsm/kg.

Compositions may include one or more buffers. Typical buffers include: a phosphate buffer; a Tris buffer; a borate buffer; a succinate buffer; a histidine buffer (particularly with an aluminum hydroxide adjuvant); or a citrate buffer. Buffers will typically be included in the 5-20mM range.

The composition may include material for a single administration, or may include material for multiple administrations *(i.e.* a 'multidose' kit). The inclusion of a preservative is preferred in multidose arrangements. As an alternative (or in addition) to including a preservative in multidose compositions, the compositions may be contained in a container having an aseptic adaptor for removal of material.

Compositions suitable for use in humans in accordance with the invention are typically administered in a dosage volume of about 0.5ml, although a half dose (*i.e.* about 0.25ml) may be administered to children.

As well as methods of treatment described herein, the present disclosure also describes a nucleic acid sequence encoding a TERT for use in therapy. The disclosure also describes a nucleic acid vector comprising a coding sequence for telomerase reverse transcriptase (TERT), for use in a method of therapy and a gene therapy vector comprising a coding sequence for telomerase reverse transcriptase (TERT), for use in a method of therapy. In particular, the therapy may be treating or ameliorating an age-related disorder.

As described for the disclosed methods of treatment, the TERT nucleic acid sequence may be the sequence as recited in SEQ ID NO: 1 or SEQ ID NO: 3 or a fragment or functional equivalent thereof. The TERT protein may have a sequence as recited in SEQ ID NO: 2 or SEQ ID NO: 4, or a fragment or functional equivalent thereof.

In the present disclosure, all of the technical and scientific terms are of the same meaning as that commonly understood by an expert in the field to which the invention pertains. Throughout the description and the claims, the word "comprises" and the variations thereon are not intended to exclude other technical features, components or steps. For the person skilled in the art, other objects, advantages and characteristics of the invention will be implied in part from the description and in part from the practice of the invention.

The invention will now be described in detail, with reference to the examples and accompanying drawings. The following examples and drawings are provided for illustrative purposes and are not intended to be limiting of the present invention.

### BRIEF DESCRIPTION OF THE DRAWINGS

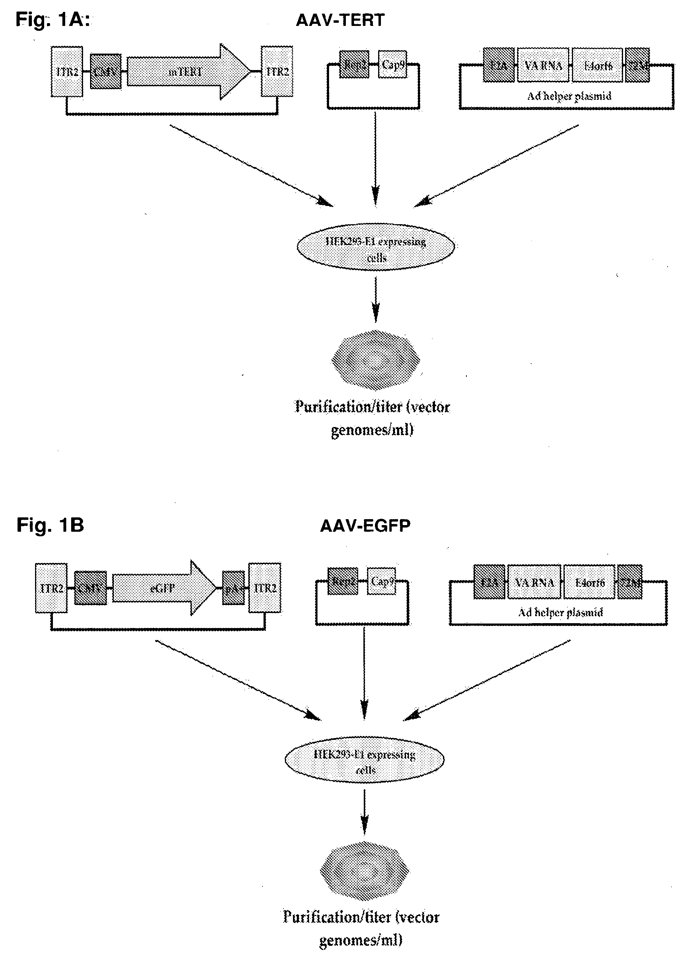

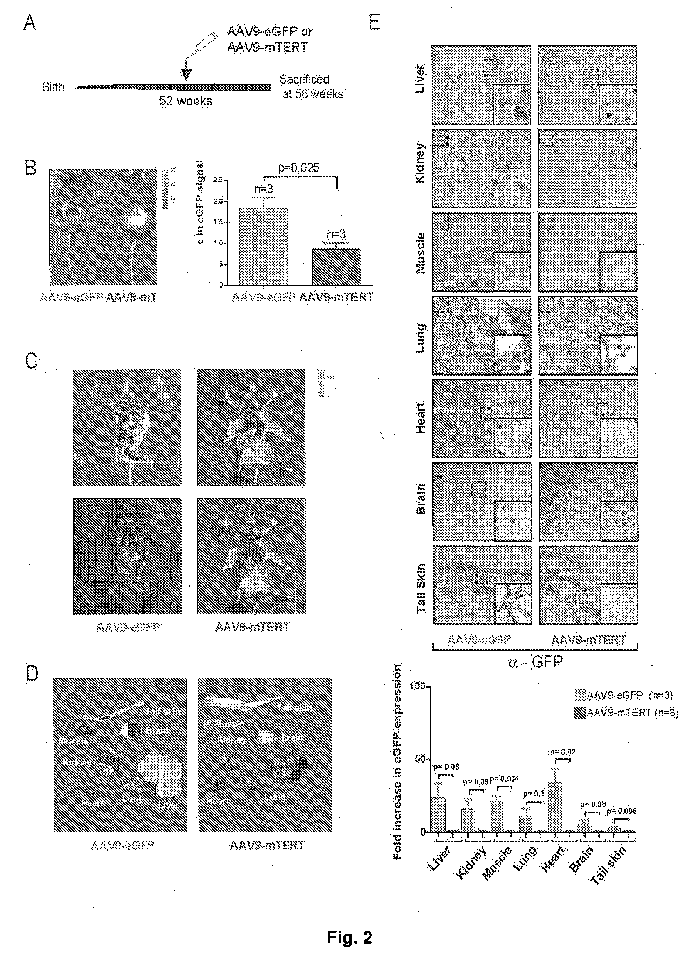

**Fig. 1****: Protocol for obtaining recombinant adenoviral vectors containing the telomerase coding sequence (****Fig. 1A****) or the green fluorescent protein coding sequence (****Fig. 2B****).** Vectors were obtained by a triple transfection protocol, where HEK293 cells expressing adenoviral E1 proteins were transfected with: a cassette containing the desired coding sequence (mTERT: mouse telomerase reverse transcriptase, or eGFP: enhanced green fluorescent protein) operatively linked to the human cytomegalovirus promoter (CMV) and with the SV40 polyadenylation signal (pA+) attached at 3' in GFP vectors, while 3' UTR from TERT was kept as polyA signal, flanked at both ends by the inverted terminal repeats of serotype 2 adeno-associated viruses (ITR2); b) a plasmid capable to express the rep protein of serotype 2 adeno-associated viruses (Rep2) and the capsid proteins of serotype 9 adeno-associated viruses (Cap9); c) a plasmid capable to express the remaining adenoviral proteins essential for the assembly of viral particles ("Ad helper plasmid"). Upon completion of the viral cycle, viral particles were purified and titered.
**Fig. 2****: Viral transduction efficiency. A.** Scheme showing the design of the experiment of administration. **B.** Direct GFP measurements in the shaved back skin of mice (n=3) treated with either AAV9-eGFP and AAV9-mTERT vectors. **C,D.** Direct GFP measurements in the indicated organs from mice (n=3) treated with either AAV9-eGFP and AAV9-mTERT vectors. **E.** Immunohistochemistry with an antibody recognizing GFP of tissues from either AAV9-eGFP or AAV9-mTERT treated mice. Quantification from at least 2 independent mice was used for the analysis.

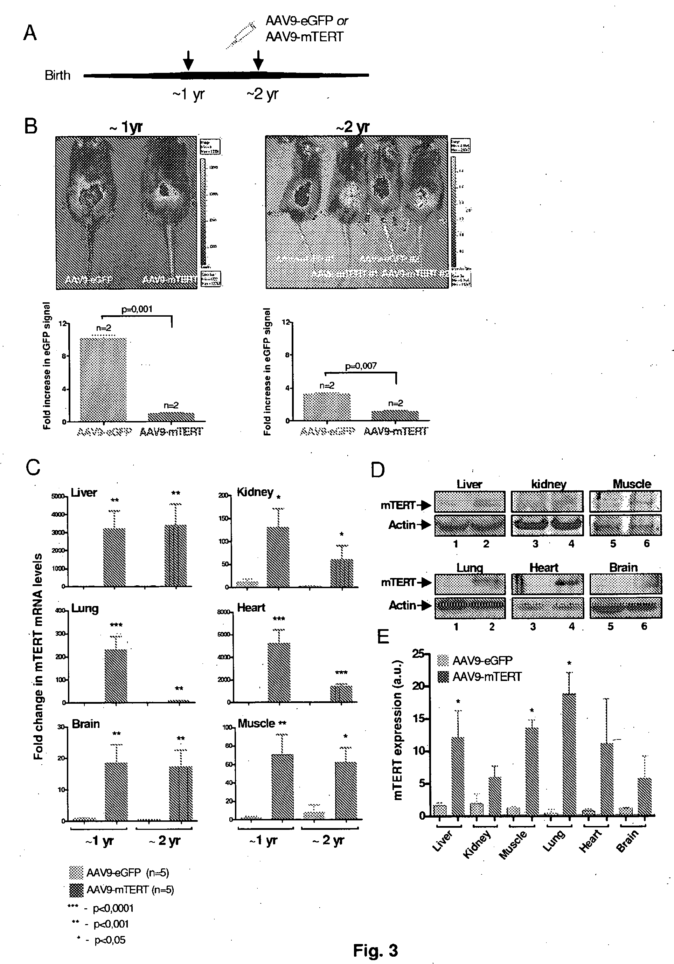

**Fig. 3****: AAV9-mediated mTERT expression in adult and in old mice: A.** Scheme of AAV9 treatments at 1 year and 2 years of age. **B.** Direct GFP measurements in the shaved back skin of AAV9-mTERT or AAV9-eGFP-treated mice. GFP expression in AAV9-mTERT treated mice was set to 1. Two mice were used per group. **C.** Fold change in mTERT mRNA levels in AAV9-mTERT treated mice with respect to mTERT mRNA expression in age-matched AAV9-eGFP controls. mTERT mRNA values are corrected by actin levels. At least 5 mice per group were used for the analysis. **D.** Whole cell extracts of the indicated tissues from the 1 year-old group were blotted for the presence of mTERT using a commercial antibody. Upper panel, representative Western blot. Bottom panel, quantification of mTERT protein levels from at least 2 independent mice. Values are normalized to actin. Student's t test was used to assess statistical significance between age-matched groups

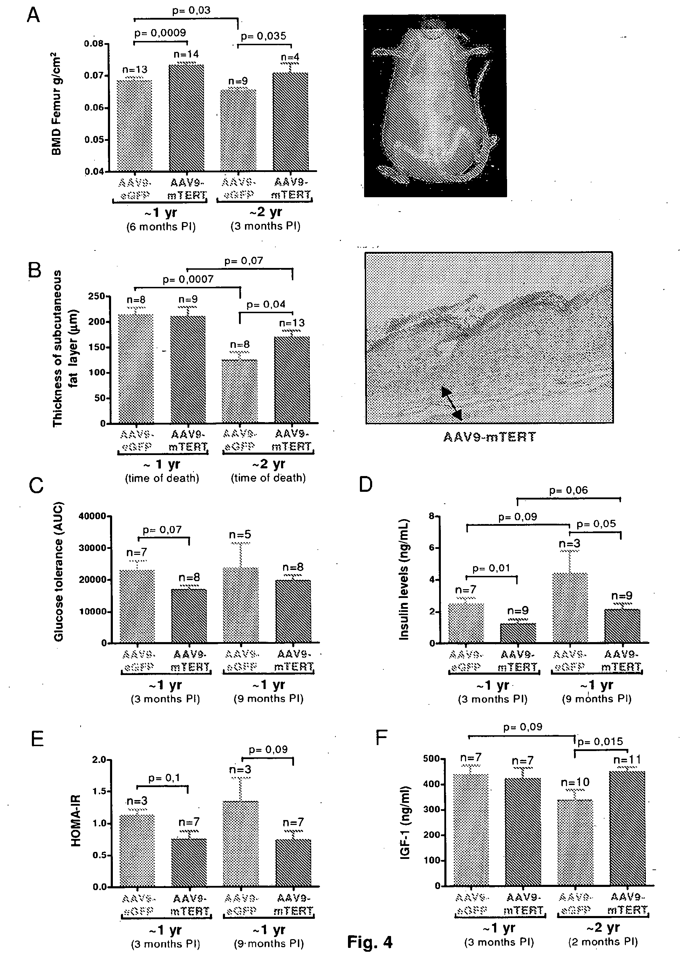

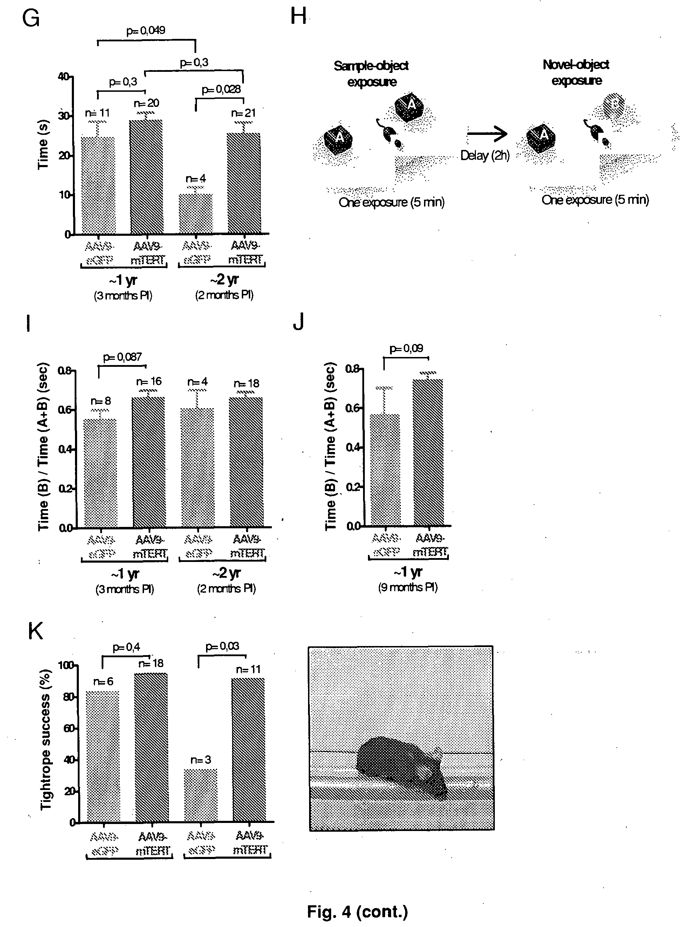

**Fig. 4****. Delayed ageing in AAV9-mTERT treated mice. A.** Bone mineral density of the femur (BMD femur) at the indicated times post-treatment with either AAV9-mTERT or AAV9-eGFP vectors. **B.** Thickness of the subcutaneous fat layer at time of death in AAV9-mTERT and AAV9-eGFP treated mice. **C.** Glucose tolerance measured as the area under the curve (AUC) at the indicated times post-treatment with either AAV9-mTERT or AAV9-eGFP vectors. **D.** Insulin levels at the indicated times post-treatment with either AAV9-mTERT or AAV9-eGFP vectors. **E.** Homeostatic model assessment score was measured as described in (Matthews, Hosker *et al.* 1985; Bonora, Targher *et al.* 2000). AAV9-mTERT treated mice show a better score at different time points post treatment. **F.** IGF-1 levels at the indicated times post-treatment with either AAV9-mTERT or AAV9-eGFP vectors. **G.** Coordination and balance was quantified as the time spent at the Rota-Rod wheel, with a constant acceleration protocol. Three trials were measured per mice. **H.** Representative image showing the design of the object recognition test. **I**. Object recognition test was performed at the indicated times post-treatment with either AAV9-mTERT or AAV9-eGFP vectors. Results correspond to the ratio of time spend in the new object *versus* the total time spent with both objects. **J.** Object recognition test was performed at the indicated times post-treatment with either AAV9-mTERT or AAV9-eGFP vectors. **K.** Neuromuscular coordination was quantified as the percentage of mice that pass with success the tightrope test at the indicated times post-treatment with either AAV9-mTERT or AAV9-eGFP vectors. Student's t test was used to assess significance between age-matched groups.

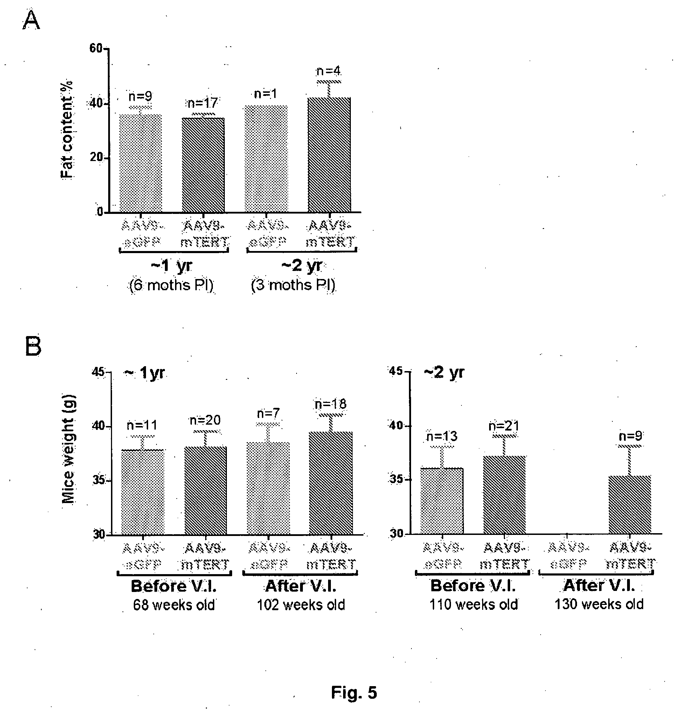

**Fig. 5****: Fat content and body weight. A.** Fat content of mice treated with either AAV9-eGFP or AAV9-mTERT vectors at the indicated times post-treatment. **B.** Body weight of the indicated mice is given as mean± SEM.

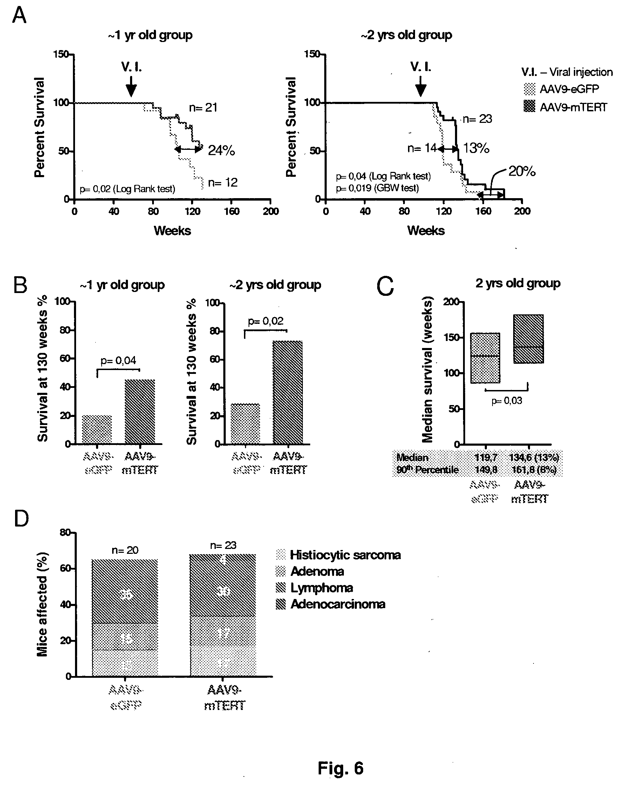

**Fig. 6****. Increased median and maximum longevity of AAV9-mTERT treated mice. A.** Survival curves of the indicated mice groups. Mice alive still are plotted as a vertical line. Statistical significance was assessed with the Log rank test (1 and 2 years old groups) as well as the Gehan-Breslow-Wilcoxon Test (2 year old group). **B.** Survival at 130 weeks. Statistical significance was calculated using the Chi² test. **C.** Average and 90^{th} percentile lifespan of 2 year-old mice treated with either AAV9-mTERT or AAV9-eGFP vectors. Statistical significance was calculated using the Student's *t* test. **D.** Percentage of mice with the indicated malignant tumour at their time of death.

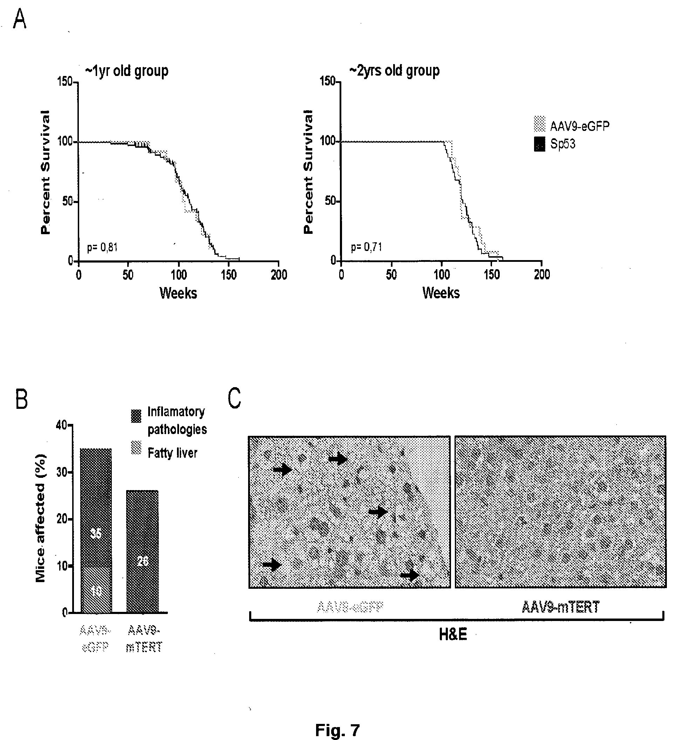

Fig. 7. Negligible **contribution of AAV9-eGFP treatment to mouse survival. A.** Survival curves (Kaplan-Meier representation) of AAV9-eGFP-treated mice and of Sp53 mice (Tomas-Loba, Flores *et al.* 2008). A Log rank test indicated lack of statistically significant differences between curves. **B.** Percentage of mice showing the indicated degenerative lesions at their time of death. 10% of the AAV9-eGFP-treated mice presented a fatty liver phenotype, which was not present in the AAV9-mTERT treated mice. **C.** Representative images of fatty liver. Arrows point to fat droplets characteristic of the fatty liver condition. Liver sections were stained with H&E

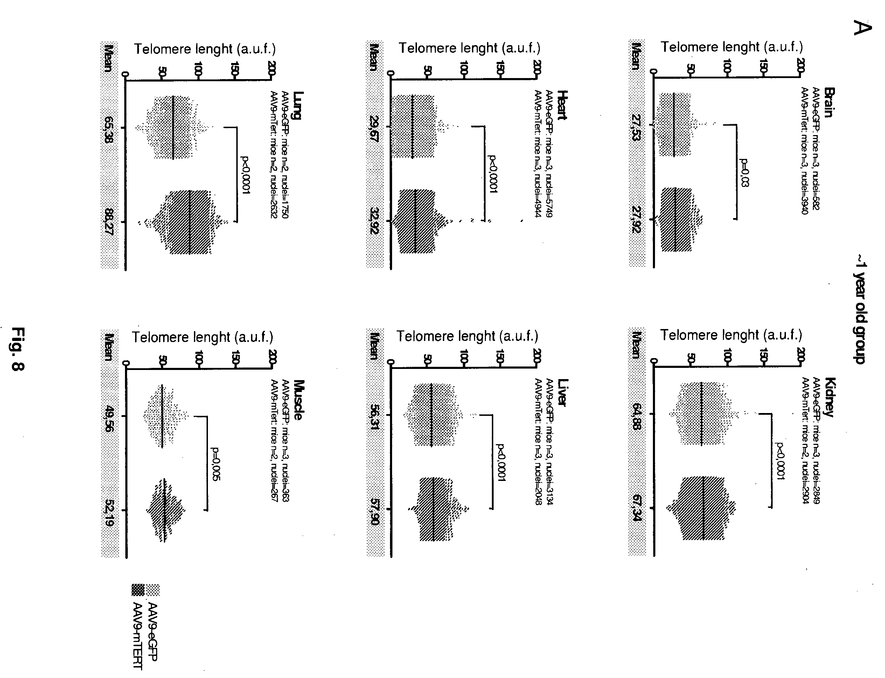

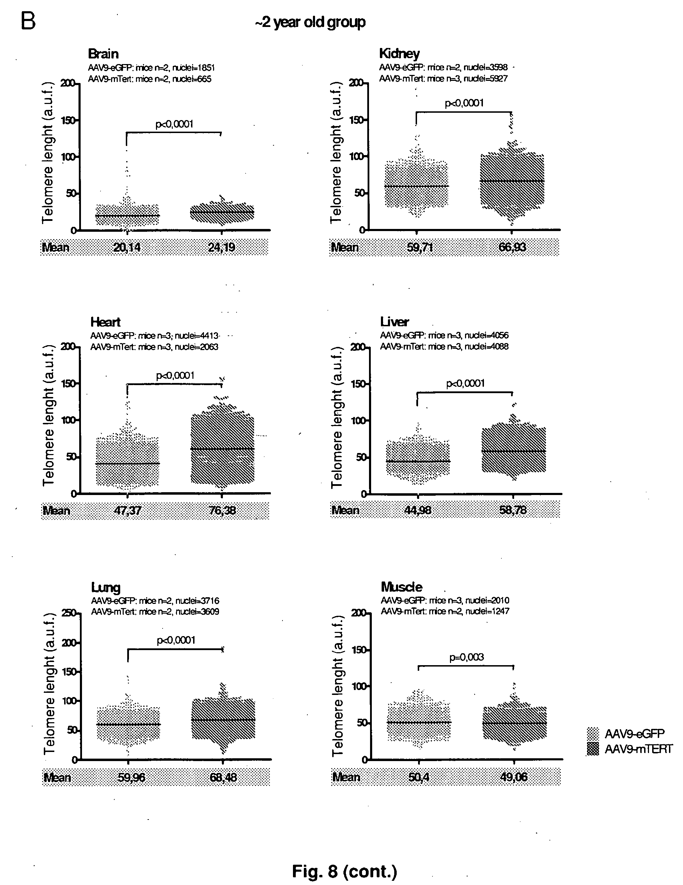

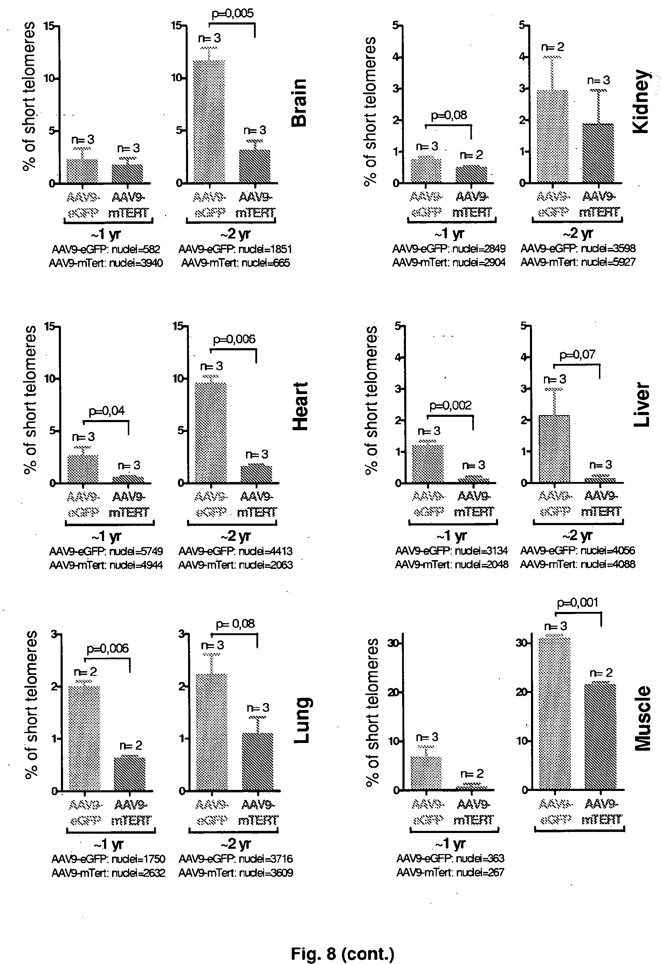

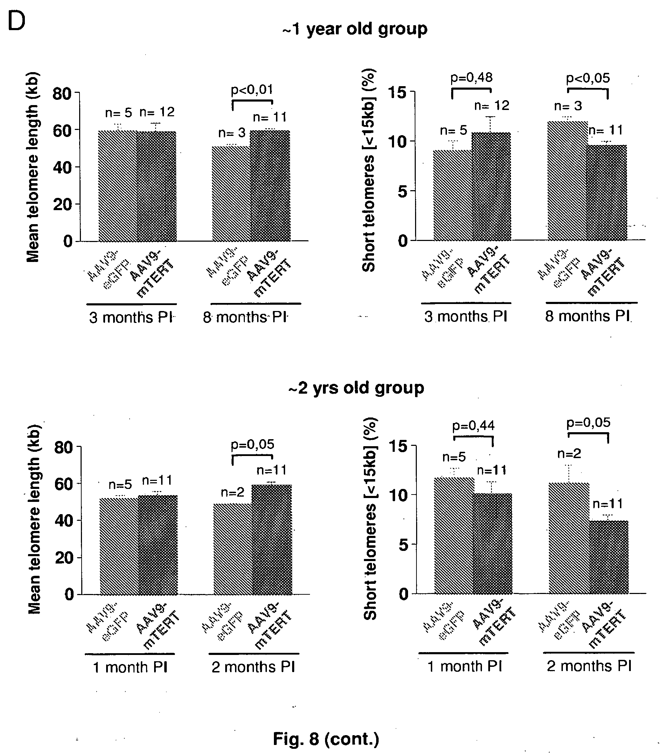

**Fig. 8****. AAV9-mTERT treatment results in increased telomere length and decreased abundance of short telomeres. A,B.** Telomere length as determined by Q-FISH of the indicated tissues from the 1 year old (A) and 2 year old (B) groups 1 month after treatment with either AAV9-mTERT or AAV9-eGFP vectors. Data are given as mean±SEM. **C.** Percentage of short telomeres (fraction of telomeres presenting an intensity below 50% of the mean intensity) 1 month after treatment with either AAV9-mTERT or AAV9-eGFP vectors. Data are given as mean±SEM. **D.** Average telomere length as determined by HT-QFISH in white blood cells at the indicated times post-treatment with either AAV9-mTERT or AAV9-eGFP vectors. The fraction of telomeres presenting less than 5Kb (corresponding to short telomeres) is shown in the right panel. Student's t test was used to assess significance between groups.

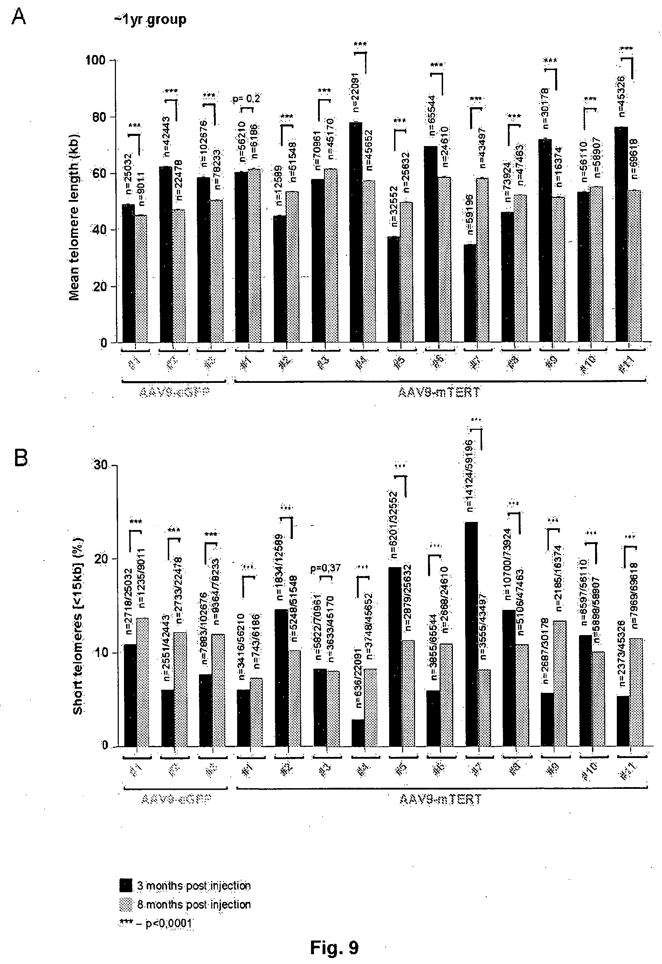

**Fig. 9****: Increase telomere length and decreased abundance of short telomeres in AAV9-mTERT mice treated mice. A.** Mean telomere length as determined by HT-QFISH in white blood cells extracted from the same mouse at different times post-treatment with the indicated AAV9 vectors. Results are presented per individual mouse (#1-11). **B.** Percentage of telomeres shorter than 15Kb is shown for the same mice. Student's *t* test was used to assess statistical significance between groups.

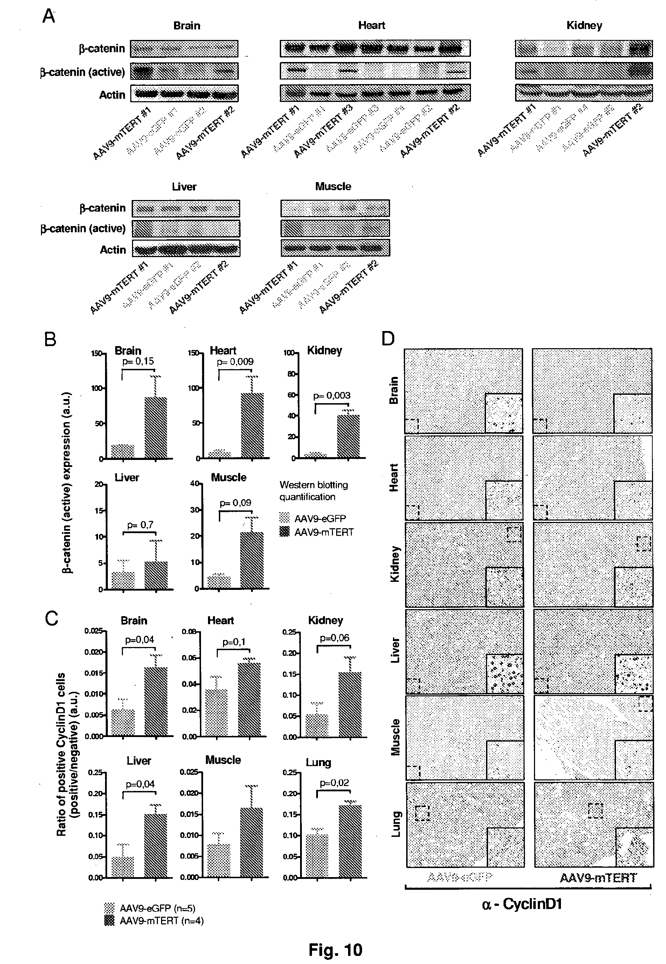

**Fig. 10****: Tissues from AAV9-mTERT treated mice show an increased expression of the active form of β-catenin and a higher percentage of cyclin D1-positive cells. A.** Representative Western blot analysis (β-catenin, active β-catenin, and actin) of whole extracts from the indicated tissues from 2 year old mice treated with either AAV9-mTERT or AAV9-eGFP vectors, 1 month post-treatment. **B.** Quantification of active - catenin in Western blots. Values are corrected by actin levels. Data are mean intensity±SEM. **C.** Quantification of CyclinD1-positive cells with respect to total cells scored (between 2x10⁵ and 1x10⁷ cells per mouse, depending on the tissue) in the indicated tissues from mice treated with either AAV9-mTERT or AAV9-eGFP vectors, 1 month post-treatment. Student's t test was used for statistical assessments. **D.** Representative CyclinD1 immunohistochemistry images of different tissues from mice treated with either AAV9-mTERT or AAV9-eGFP vectors, 1 month post-treatment.

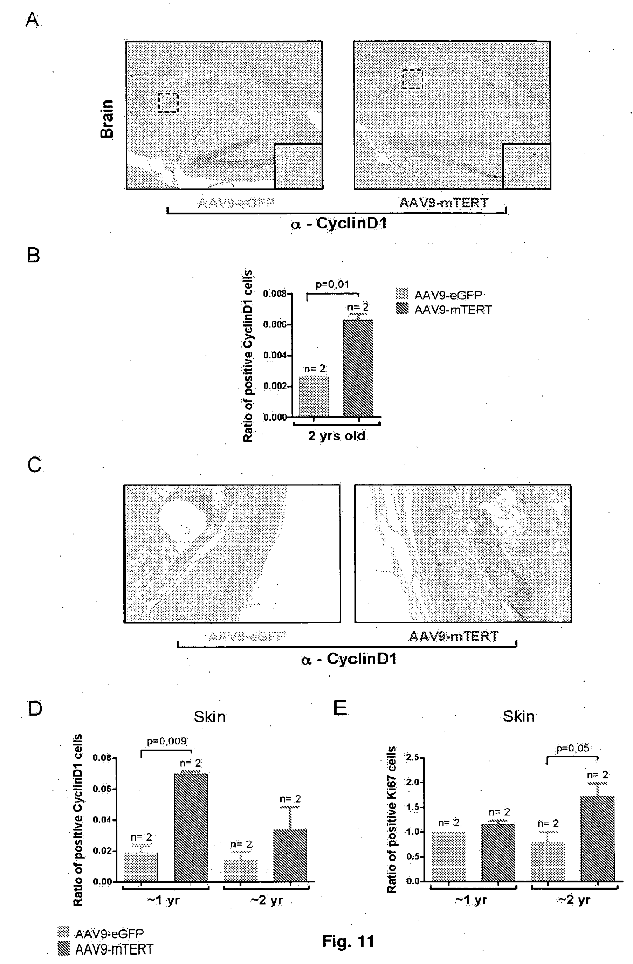

**Fig. 11****. Increased CyclinD1 expression in tissues from AAV9-mTERT** treated **mice. A.** Representative images of CyclinD1 staining of the hippocampus of 2 year old mice treated with either AAV9-eGFP and AAV9-mTERT vectors. **B.** Quantification of CyclinD1-positive cells from two independent experiments. **C.** Representative images of CyclinD1 staining of the skin of 2 year old mice treated with either AAV9-eGFP and AAV9-mTERT vectors. **D.** Quantification of CyclinD1-positive cells out of total number of cells scored (between 1x10⁵ and 3x10⁶ cells per mouse) in age-matched AAV9-mTERT or AAV9-eGFP treated mice. Student's *t* test was used for statistical assessments. **E.** Ratio of Ki67 positive cells out of the total number of cells scored (between 2x10⁴ and 8x10⁴ cells were scored per mouse) in skin sections from AAV9-eGFP or AAV9-mTERT treated mice. Student's *t* test was used for statistical calculations.

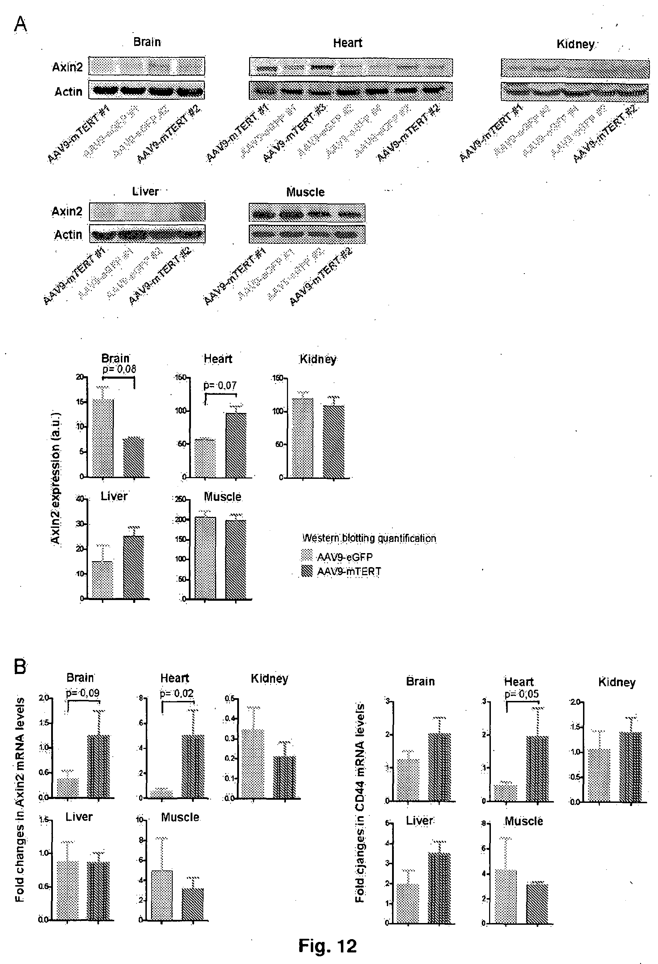

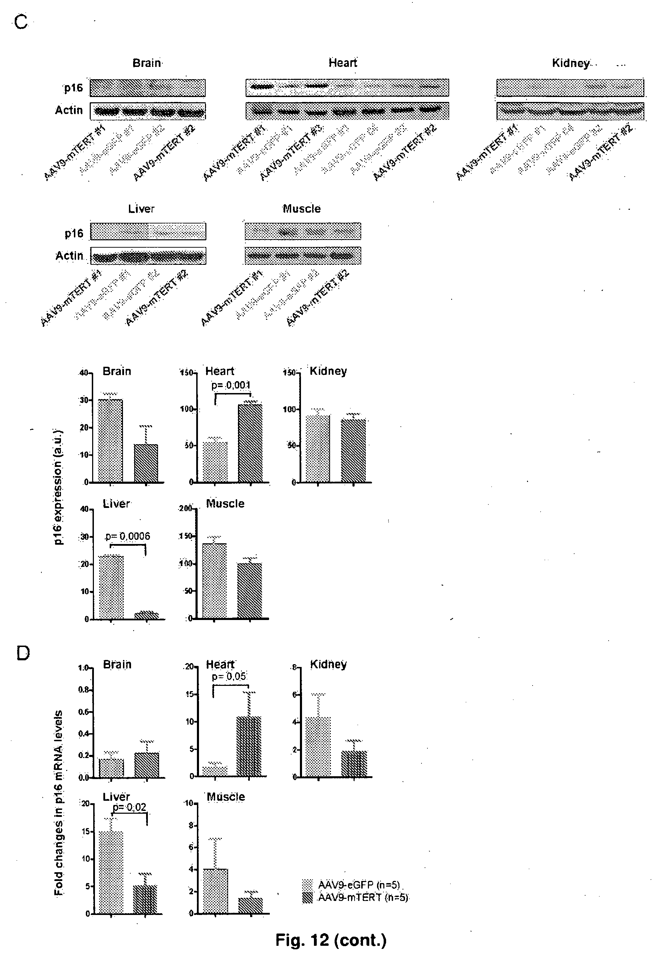

**Fig. 12****. Effect of AAV9-mTERT treatment on Wnt targets. A.** Representative Western blot (Axin2, Actin) of the indicated tissues from 2 year old mice treated with either AAV9-mTERT or AAV9-eGFP vectors. Quantification of Axin2 expression is shown in the bottom panel. Values were corrected by actin and correspond to mean intensity±SEM. **B.** mRNA levels of Wnt target genes Axin2 (left panel) and CD44 (right panel) in mice treated with either AAV9-mTERT or AAV9-eGFP vectors. Values are normalized for actin and are represented as the mean of 2 ^{Ct} values relative to samples of age-matched AAV9-eGFP mice (n=5 for each group). **C.** Representative Western blot (p16, Actin) of the indicated tissues from 2 year old mice treated with either AAV9-mTERT or AAV9-eGFP vectors. Quantification of p16 expression is shown in the bottom panel. Values were corrected by actin and correspond to mean intensity±SEM. **D.** Expression of p16 mRNA in the indicated tissues from mice treated, with either AAV9-mTERT or AAV9-eGFP vectors. Data was normalized for actin and are represented as the mean of 2 ^{Ct} values relative to samples of age-matched AAV9-eGFP mice (n=5 for each group). Student's *t* test was used to assess significance between tested groups.

### EXAMPLES

The Examples set forth below were carried out using the following materials and techniques:

### Mice

Mice were produced at a specific pathogen-free barrier area of the CNIO, in accordance with the recommendations of the Federation of European Laboratory Animal Science Associations. Mice were maintained in the original 100% C57BL6 background. Separate groups of mice were tail-vein injected with 2x10^12 vg/animal of either AAV9-GFP or AAV9-mTERT at 360 days (AAV9-GFP n=11, AAV9-mTERT n=20) or 720 days (AAV9-GFP n=14, AAV9-mTERT n=23) of age.

Mice were daily inspected by an authorized animal facility technician and sacrificed when they presented signs of morbidity or tumours in accordance to the Guidelines for Humane Endpoints for Animals Used in Biomedical Research (Harrison, Strong *et al.* 2009). The date of euthanasia was used as an estimation of the mouse lifespan. All mice were subjected to necropsy and histopathological analysis.

### Histological analysis and immunohistochemistry

Histopathology was performed as previously described (Gonzalez-Suarez, Samper *et al.* 2001). Briefly, mouse tissues and organs were fixed overnight in a 10% neutral-buffered formalin solution at 4°C, dehydrated through graded alcohols and xylene, and embedded in paraffin. Histological analysis was performed on 4-5 µm sections according to standard procedures. Cancer related pathologies were grouped in four types as described (Tomas-Loba, Flores *et al.* 2008).

Full histopathological analysis was performed by our veterinary and the degenerative pathologies were grouped as previously described.

Immunohistochemistry was performed on deparaffinated tissues and processed with hematoxylin and eosin (H&E) or the indicated antibodies: Anti-GFP (Invitrogen, A11122), Anti-CyclinD1 (Thermo Scientific, RM-9104, Clone SP4), Anti-Phospho-Smad2 (SER465/467, Chemicon AB3849) and Anti Ki-67 (DakoCytomation, M7249, clone TEC-3).

### Recombinant AAV vectors

Recombinant adeno-associated virus-based vectors (AAV) were generated following the procedure shown in Fig. 1, common for both the vectors containing the telomerase coding sequence (AAV-TERT) and control vectors containing the an enhanced green fluorescent protein coding sequence (AAV-GFP). Both AAV vectors have been made by a triple transfection protocol of HEK293 cells, which cells are capable to complement the E1 adenovirus proteins, whose coding sequences are absent from the plasmid of the helper adenovirus. The protocol was performed according to standard methods (Matsushita 1998 p938). The following elements were used: a) the cassette of interest with the backbone of the genome of the recombinant adenoviral vectors, comprising the coding sequence of the gene of interest operatively linked to a promoter flanked by ITRs from serotype 2, b) the plasmid containing AAV genes, which can be changed according to the desired serotype: in all plasmid rep genes are from serotype 2 and only the capsid is from the serotype that is intended to use, c) essential adenoviral genes are provided in the third plasmid as it is always the same.

The expression cassettes used were (i) GFP (green fluorescent protein) under the control of CMV promoter and SV40 polyA signal or (ii) murine TERT (coding sequence: SEQ ID NO:1) under the control of CMV promoter. In AAV-TERT vectors, SV40 polyA was not used since 5'UTR from TERT was maintained as a polyA signal. The reason for that is that murine TERT is >3kb and the maximal size for correct encapsidation including ITR+promoter+gene+polyA is 4.7kb. Thus, the mTERT sequence does not have any external polyA. Otherwise the vector was oversized.

HEK293 cells were cultured in roller bottles (Corning, New York, USA) in DMEM 10% FBS to 80% confluence and co-transfected with a plasmid carrying the expression cassette flanked by the AAV2 viral ITRs, a helper plasmid carrying the AAV *rep2* and *cap9* genes (kindly provided by K.A. High, Children's Hospital of Philadelphia), and a plasmid carrying the adenovirus helper functions (kindly provided by K.A. High, Children's Hospital of Philadelphia).. AAV vectors were purified with an optimized method based on two consecutives cesium chloride gradients, dyalized against PBS, filtered and stored at -80°C until use (Ayuso *et al* Gene therapy 2010). The titers of viral genomes particles were determined by quantitative real time PCR

### Measurement of virus transduction efficiency

A control group of 100% C57BL6 background mice (AAV9-GFP n=5, AAV9-mTERT n=5) was injected at 360 days of age following the methodology described before. At 4 weeks post-injection mice were sacrificed and subjected to either pathological analysis or GFP and mTERT expression assessments. GFP expression from skin and internal organs was determined using a 488 nm excitation light and image collecting system (IVIS Imageing System 200 Series, Xenogen Corporation, Alameda, CA). Telomerase expression in different tissues was analyzed by western blot analysis and quantitative RT-PCR.

### Bone density

Bone mineral density was measured in anesthetized living animals, or post-mortem when referred, using a Dual Energy X-ray Absorptiometry (DEXA) scan device. Bone mineral density (BMD) indicates the density of minerals in mice bones.

### Skin measurements

Subcutaneous fat layer measurements were performed as described in (Tomas-Loba, Flores *et al.* 2008). Briefly, 10 to 20 random measurements along the skin from at least 5 mice (3 skin sections per mice) per injected group were considered. Skin sections cut perpendicular to the skin surface at a 5 mm thickness were stained for hematoxylin-eosin (H&E), and Image J software was used for length measurements.

### Intraperitoneal Glucose Tolerance Tests

Glucose curves were performed as described elsewhere (Moynihan, Grimm *et al.* 2005). Briefly, mice were fasted for at least 8 hr, and injected intraperitoneally with a 50% dextrose solution (2 g/kg body weight). Blood glucose levels were measured with a glucometer (Glucocard Memory 2, Arkray, Japan) at the indicated times after injection. Data analysis was performed as described in (Tomas-Loba, Flores *et al.* 2008).

Blood insulin levels were measured with Ultra-Sensitive Mouse Insulin ELISA Kit (Crystal Chem Inc. #90080), following manufacturers protocol.

### Fat content

Fat content was measured with the same procedure described for bone mineral density acquirements, using the DEXA scan.

### Telomere O-FISH analysis on paraffin sections

Q-FISH determination on paraffin-embedded tissue sections from 1-year and 2-year old mice were hybridized with a PNA-telomeric probe, and fluorescence intensity of telomeres was determined as described (Gonzalez-Suarez, Samper *et al.* 2001). Quantitative image analysis was performed using the Definiens Developer Cell software (version XD 1.2; Definiens AG). For statistical analysis a two-tailed Student t test was used to assess significance (GraphPad Prism software).

### Quantitative real-time RT-PCR

Total RNA from tissues was extracted with Trizol (Life Technologies). RNA samples were *DNase I* treated, and used as template for a reverse transcription reaction using random primers and Superscript Reverse Transcriptase (Life Technologies), according to the manufacturer's guidelines. Quantitative real-time PCR was performed using an ABI PRISM 7700 (Applied Biosystems), using DNA Master SYBR Green I mix (Applied Biosystems).

The primers used were:
*Actin-For:* GGCACCACACCTTCTACAATG (SEQ ID NO:7);
*Actin-Rev:* GTGGTGGTGAAGCTGTAG (SEQ ID NO:8);
*TERT-For:* GGATTGCCACTGGCTCCG (SEQ ID NO:9);
*TERT-Rev:* TGCCTGACCTCCTCTTGTGAC (SEQ ID NO:10).
*p16-For:* CGTACCCCGATTCAGGTGAT (SEQ ID NO: 11)
*p16-Rev:* TTGAGCAGAAGAGCTGCTACGT (SEQ ID NO:12)
*Axin2*-*For:* GGCAAAGTGGAGAGGATCGAC (SEQ ID NO:13)
*Axin2-Rev:* TCGTGGCTGTTGCGTAGG (SEQ ID NO:14)
Cyclin D1 - For: TGCGCCCTCCGTATCTTAC (SEQ ID NO:15)
Cyclin D1 - Rev: ATCTTAGAGGCCACGAACATGC (SEQ ID NO:16)
CD44 - For: CAGCCTACTGGAGATCAGGATGA (SEQ ID NO: 17)
CD44 - Rev: GGAGTCCTTGGATGAGTCTCGA (SEQ ID NO:18)
Klf4 - For: GCGAACTCACACAGGCGAGAAACC (SEQ ID NO: 19)
Klf4 - Rev: TCGCTTCCTCTTCCTCCGACACA (SEQ ID NO:20)
Tieg1 - For: CCCATTGCCCCTGCTCCTG (SEQ ID NO:21)
Tieg 1 - Rev: TGTGTCCGCCGGTGTCTGG (SEQ ID NO:22)

Statistical analyses (Student's t-test) were performed on the Ct values as described before (Munoz, Blanco *et al.* 2005).

### Western blots

Western blots were made on whole cell extracts from the indicated tissues. Tissues from moribund animals were collected and immediately snap frozen post-mortem. The antibodies used were: Anti-h/mTERT (Calbiochem, 582005), Anti- -Catenin (BD Laboratories, 610154), Anti- Active-β-Catenin (Millipore, 05-665), Anti-Axin2 (Abcam, 32197), Anti-TGFα (Abcam, 66043), Anti-Smad3 (Abcam, 28379), Anti-p16 (Santa Cruz, 1207), Anti-β-Actin (Sigma, A5441) and Anti-Tubulin (Abcam, 6161). The quantification was made with Scion Image Software.

### Neuromuscular coordination and object recognition assays

The object recognition memory test was carried out as explained before (Bevins and Besheer 2006) and briefly explained in **Fig. 2h****.** To evaluate the motor coordination and balance, mice were tested in a Rota-Rod apparatus (model LE 8200); the measures score the latency of fall, with a continuous acceleration protocol, from 4 to 40 rpm, for a maximum of 60s in three trials (Castelhano-Carlos, Sousa *et al.* 2009). The tightrope test was performed as described elsewhere (Ingram and Reynolds 1986; Matheu, Maraver *et al.* 2007; Tomas-Loba, Flores *et al.* 2008)

### Example 1: AAV9 mediated expression of mTERT leads to a stable and efficient systemic transduction of mouse tissues

As explained, the present inventors generated an adeno-associated virus (AAV) carrying the mouse TERT (mTERT) cDNA (SEQ ID NO:1) under the control of the CMV promoter and containing the capsid proteins of the AAV serotype 9, known to confer tropism for a wide variety of mouse tissues (AAV9-mTERT). As a negative control and reporter of AAV9 expression, an analogous construct containing the enhanced green fluorescent protein (eGFP) instead of mTERT was generated (AAV9-eGFP).

First, to assess viral tropism in the experimental setting, a small group of 1-year old mice were sacrificed 1 month post-injection with either AAV9-mTERT or AAV9-eGFP vectors **(****Fig. 2****, panel A).** AAV9-eGFP injected mice showed increased eGFP signal in the shaved back skin compared to AAV9-mTERT injected mice as detected by direct eGFP fluorescence **(****Fig. 2****, panel B).** Upon sacrifice, analysis of internal organs by direct eGFP fluorescence **(****Fig. 2****, panels C and D)** or by immunohistochemistry (IHC) with anti-eGFP antibodies **(****Fig. 2****, panel E),** showed a significant increase in eGFP expression in AAV9-eGFP mice compared to AAV9-mTERT cohorts, which varied depending on the target tissue. In agreement with the known tropism of AAV9, liver, kidney, muscle, heart and lung were the preferential target tissues and, to a lesser extent, significant eGFP expression in the brain and skin was found (Inagaki, Fuess *et al.* 2006; Zincarelli, Soltys *et al.* 2008).

For longevity and ageing studies, AAV9-mTERT or AAV9-eGFP vectors were injected via tail vein (2x10¹² vg/mouse) into two large cohorts of 68 and 110 weeks of age (referred hereafter as 1 and 2 year old groups; see **Fig. 3****, panel A),** and efficient viral infection was confirmed by direct assessment of eGFP fluorescence in living animals at 2 weeks post-infection **(****Fig. 3****, panel B).** One month post-infection, TERT mRNA and protein levels were significantly increased in several tissues from AAV9-mTERT injected mice compared to AAV9-eGFP controls **(****Fig. 3****, panels C,D),** including liver, kidney, lung, heart, brain and muscle. Although the apparent fold increase in TERT mRNA levels was overbearing, mTERT protein levels were increased by 5-20 fold depending on the target tissue **(****Fig. 3****, panel E).** This level of mTERT protein over-expression achieved by using gene therapy vectors is in the same range of mTERT over-expression reached by classical mouse transgenesis, including the long-lived Sp53/Sp16/SARF/TgTERT mouse model (Gonzalez-Suarez, Samper *et al.* 2001; Gonzalez-Suarez, Flores *et al.* 2002; Flores, Evan *et al.* 2006; Tomas-Loba, Flores *et al..* 2008).

Together, these results demonstrate that AAV9 can be used as a strong and reliable in *vivo* transducer of genes, which allows a significant increase in mTERT protein expression in a wide range of mouse tissues at the desired time in the lifespan of mice.

### Example 2: mTERT ectopic expression late in life decreases the incidence of age-related osteoporosis and glucose intolerance

Bone loss is a well-characterized sign of the ageing process both in mice and humans (Parfitt 1984; Ferguson, Ayers *et al.* 2003), which results from bone resorption due to osteoblast insufficiency. As shown in **Fig. 4****, panel A,** 3-6 months after AAV9 treatment, the mineral density of the femur (BMD femur) was significantly higher in the AAV9-mTERT treated mice of both age groups compared to the age-matched AAV9-eGFP treated controls, suggesting a beneficial effect of increased TERT expression in preventing osteoporosis associated with mouse ageing. As expected, a significant decrease in femur BMD was observed in the 2 year-old control group compared to the 1 year old controls **(****Fig. 4****, panel A).**

A well-established biomarker of ageing is the loss of the subcutaneous adipose skin layer, which in turn can lead to opportunistic pathologies associated with old age such as impaired wound-healing and infections (Steen 1988; Shimokata, Tobin *et al.* 1989; de Boer, Andressoo *et al.* 2002; Tomas-Loba, Flores *et al.* 2008). Accordingly, we observed a significant decrease in the thickness of the subcutaneous fat layer in the 2 year-old control group compared to the 1 year-old controls **(****Fig. 4****, panel B).** Remarkably, this decrease did not occur in the AAV9-mTERT treated mice, instead, we observed a better preservation of subcutaneous fat layer in AAV9-mTERT treated mice of the 2-year old group compared to the aged-matched AAV9-eGFP treated controls **(****Fig. 4****, panel B).**

Glucose intolerance and insulin resistance are also well-established indicators of the ageing progression (Bailey and Flatt 1982; Guarente 2006). AAV9-mTERT treated mice of the 1 year old group showed a faster glucose uptake at both 3 and 9 months post-treatment compared to the age-matched AAV9-eGFP treated controls **(****Fig. 4****, panel C).** Furthermore, significantly lower levels of insulin in the AAV9-mTERT treated mice of both age groups suggest an improved insulin sensitivity compared with the AAV9-eGFP treated controls, which presented increased insulin levels with ageing **(****Fig. 4****, panel D).** The better glucose uptake and the improved insulin sensitivity of AAV9-mTERT treated mice is reflected by an overall improvement of the homeostatic model assessment (HOMA) score 9 months after treatment compared with control AAV9-eGFP treated mice **(****Fig. 4****, panel E)** (Matthews, Hosker *et al.* 1985; Heikkinen, Argmann *et al.* 2007). Importantly, improved glucose tolerance and low insulin levels in AAV9-mTERT treated mice were accompanied by high IGF-1 levels in the 2 year old AAV9-mTERT treated mice compared to age-matched controls, which showed a further decrease in IGF-1 levels compared to the 1 year old controls **(****Fig. 4****, panel F).** As a decline in IGF-1 expression is known to occur with increasing age (Hammerman 1987), the maintenance of high IGF-1 levels in the 2 year old AAV9-mTERT treated mice supports an improved health-span of these mice as the result of TERT intervention. Of note, improved glucose tolerance and low insulin levels were not accompanied by significant differences in body fat content as detected by densitometry scans or in body weight of the different mouse cohorts **(****Fig. 5****)**

### Example 3: mTERT ectopic expression late in life improves scores in neuromuscular and object-recognition tests

Next, to further assess the systemic effects of ectopic mTERT expression, we turned into a number of behavioural assays that test the capacity of mice to perform different tasks, which is progressively lost with the ageing progress. In particular, we evaluated (i) coordination and balance (Rota-Rod test), (ii) memory (Object Recognition Test), as well as (iii) neuromuscular coordination (Tightrope Test) (Ingram and Reynolds 1986; Bevins and Besheer 2006). Coordination and balance were significantly improved in the 2 year-old group 2 months post AAV9-mTERT treatment compared to age-matched controls **(****Fig. 4****, panel G).** Both the 1 year old and the 2 year old AAV9-mTERT treated groups presented better scores in the object recognition assay compared to the controls, which became almost significant in the 1 year old group **(****Fig. 4****, panel H-J).** Of note, memory improvement in the AAV9-mTERT treated 1 year old group was maintained at least 9 months post treatment **(****Fig. 4****, panel J).** Finally, we observed a very clear improvement in the neuromuscular coordination test in the 1 year old mice treated with AAV9-mTERT that was maintained for up to 11 months post treatment compared to AAV9-eGFP treated mice, which showed a decline in the ability to perform this test with age **(****Fig. 4****, panel K).** Together, these results demonstrate that mTERT expression ameliorates overall organ fitness and health=span in both age groups.

### Example 4: A TERT-based gene therapy late in life significantly extends lifespan without increasing cancer

In addition to the above described anti-ageing activity of TERT and its ability to improve healthspan in mice, TERT has been also recently demonstrated to increase median longevity of mice that are cancer resistant due to increased expression of the p53, p16 and p19ARF tumour suppressors (Sp53/Sp16/SARF/TgTERT) (Tomas-Loba, Flores *et al.* 2008). Here, we set to address whether our TERT gene therapy approach would be able not only to improve health span, but also to significantly extend longevity in mice treated at middle age (1 year old) or at old age (2 year old) without increasing cancer incidence.

Although all mice used in the study are of a >95% C57B16 genetic background, longevity comparisons are made between AAV9-mTERT and AAV9-eGFP treated mice within the same mouse cohort and age-group to avoid minimal possible differences between the groups. Analysis of the survival curves indicated a significant extension of lifespan in AAV9-mTERT treated mice compared to the controls in both age groups **(****Fig. 6****, panel A),** indicating the robustness of TERT expression in extending lifespan of different age groups. In particular, TERT expression led to an increase in survival of 24% in the 1 year-old group and of 13% in the 2 year old group (p<0,05 for both the 1 and 2 year old groups) **(****Fig. 6****, panel A).**

To dissect the effects of TERT on median and maximum longevity, we determined the percentage of animals that reached old age in both tested groups. In particular, we found a significant increase in the percentage of mice that survived for up to 130 weeks **(****Fig. 6****, panel B),** as well as in the median survival of the longest lived mice (90% percentile) **(****Fig. 6****, panel C)** in the AAV9-mTERT group compared to the controls, indicating that TERT impacts not only on median survival but also can modulate maximum longevity. Indeed, the longest-lived AAV9-mTERT treated mouse surpassed by 27 weeks the maximum longevity of the longest-live AAV9-eGFP treated control, 182 weeks compared to 155 weeks, respectively **(****Fig. 6****, panel A**), which represents a 20% increase in maximum longevity.

To test whether AAV9-eGFP treatment could have a negative effect on survival, we compared the survival curves of control AAV9-eGFP treated mice from both age groups with those obtained for Sp53 controls in Tomas-Loba *et al* (Tomas-Loba, Flores *et al.* 2008), previously used by us as reference for normal longevity **(****Fig. 7****, panel A)** (Garcia-Cao, Garcia-Cao *et al.* 2002; Matheu, Maraver *et al.* 2007). Remarkably, the survival curves of AAV9-eGFP treated mice showed a perfect fitting with those of Sp53 mice, indicating a normal longevity of AAV9-eGFP treated mice from both age groups.

A drawback of TERT over-expression in classical transgenic mice studies has been an increase in the incidence of cancer, except for the case of cancer-resistant Sp53/Sp16/SARF/TgTERT mice (Gonzalez-Suarez, Samper *et al.* 2001; Artandi, Alson *et al.* 2002; Canela, Martin-Caballero *et al.* 2004; Tomas-Loba, Flores *et al.* 2008). To address whether our gene therapy strategy to increase TERT expression late in life had any undesirable side effects, we performed a detailed pathological analysis of all mice in the study at their time of death. AAV9-mTERT treated mice of both age groups did not show a significant increase in cancer incidence compared to the AAV9-eGFP treated controls **(****Fig. 6****, panel D;** the observed 4% in adenocarcinoma incidence corresponds to a single adenocarcinoma in the lungs of a single AAV9-mTERT mouse). The lack of increased cancer in AAV9-mTERT treated mice compared to conventional TERT transgenics (Gonzalez-Suarez, Samper *et al.* 2001; Artandi, Alsori *et al.* 2002; Canela, Martin-Caballero *et al.* 2004) could be due to the fact that TERT transgenics have constitutive expression of telomerase from early embryo development, while AAV9 infection is targeted to adult tissues late in life, something which may significantly decrease the risk of developing cancer. In addition, opposite to transgenic mice, AAV vectors are non-integrative and therefore do not represent a permanent genetic modification. Thus, if certain cells start proliferating quickly, they will lose the AAV vector and TERT expression. This represents a safety mechanism to avoid over-proliferation of TERT expressing cells. Finally, the fact that AAV targets more efficiently post-mitotic tissues could also prevent tumour formation since these tissues are generally considered more resistant to develop tumours than highly proliferative tissues.

Remarkably, histopathological analyses revealed a tendency of AAV9-mTERT treated mice to show less inflammation-related pathologies as well as less metabolic syndrome-related pathologies (fatty liver) compared to the AAV9-eGFP group **(****Fig. 7****, panels B and C),** further suggesting that TERT significantly improves the healthspan of mice.

### Example 5: Telomerase expression late in life leads to overall telomere lengthening and decreased abundance of short telomeres in a wide variety of mouse tissues

Telomere attrition is a well-known indicator and a possible cause of organismal ageing (Harley, Futcher *et al.* 1990; Canela, Vera *et al.* 2007; Jiang, Schiffer *et al.* 2008; Calado and Young 2009). Telomerase activity decreases with mouse age and this accounts for the prominent telomere shortening observed at older ages (Flores, Canela *et al.* 2008). In turn, increased TERT expression can extend mouse lifespan and this is thought to occur through the canonical function of telomerase in extension of short telomeres, although recent works suggest a non-canonical role of telomerase on the Wnt signaling pathway, which could potentially impact on ageing (Park, Venteicher *et al.* 2009).

First, we set to address whether the TERT-induced organ and systemic enhancements described here are related to a better maintenance of telomeres. In particular, we measured telomere length in a variety of differentiated tissues (brain - *cerebral cortex,* heart - *ventricles,* lung, kidney - *cortex,* liver - *hepatocytes* and muscle - *fibers*), 1 month after treatment with either AAV9-mTERT or AAV9-eGFP vectors. To this end, we used quantitative telomere FISH (Q-FISH) on mouse tissue sections, which allows determining the intensity of individual telomere dots within nuclei of a given tissue (Methods) (Munoz, Blanco *et al.* 2005). In agreement with higher mTERT expression, most tissues in AAV9-mTERT treated mice of both age groups presented significantly longer telomeres than the age-matched AAV9-eGFP controls (**Fig. 8****, panels A,B**), suggesting that telomerase was efficiently elongating telomeres. To further support this, we measured the percentage of short telomeres in the same tissue sections, as short telomeres are a preferred substrate of telomerase. One month after AAV9-mTERT treatment, both age groups showed a significant decrease of the percentage of short telomeres compared to the AAV9-eGFP treated groups, which showed a further increase in short telomeres when comparing 1 year to 2 year old mice (**Fig. 8****, panel C**). Noteworthy, we observed a bigger impact of TERT expression in the 2 year-old group, again in agreement with a preference of telomerase for short telomeres. All together, these results indicate that increased TERT expression in adult tissues through a gene therapy approach results in a functional telomere extension that acts preferentially on short telomeres.

To understand better the effects of AAV9-mTERT treatment on telomere length, we performed a longitudinal study by measuring telomere length of the same individuals at 3 and 8 months post-treatment or 1 and 2 months post-treatment in the 1 year old and 2 year old groups, respectively. For this, we collected blood samples from all mice under study at different time points and determined telomere length by using a high-throughput (HT) Q-FISH technique, previously optimized for blood samples (Canela, Vera *et al.* 2007). As expected, average telomere length decreased with time in control mice from both age groups, which was concomitant with an increase of short telomeres (**Fig. 8****, panel D**). In contrast, AAV9-mTERT treated mice from both age groups showed almost no decrease in average telomere length at different times post-infection (**Fig. 8****, panel D**), concomitant with a decrease in the percentage of short telomeres with time (**Fig. 8****, panel D**). As AAV9-mTERT vectors trasduce very poorly bone marrow and spleen, there results may reflect on the better health status of AAV9-mTERT treated mice compared to the AAV9-eGFP controls, in agreement with previous studies suggesting a link between health status and maintenance of telomere length.(Ornish D, *et al.,* 2008; Lin J *et al.,* 2010).

Interestingly, when the behavior of telomeres was represented per individual mouse at two different times after treatment (3 and 8 months post infection), we observed that 7 out of 11 AAV9-mTERT treated mice of the 1 year old group showed no decrease or even a net increase in average telomere length compared to the previous time point (**Fig. 9****, panel A**). This was accompanied by 6 out of 11 mice showing no increase or even a marked decrease in percentage of short telomeres with time (**Fig. 9****, panel B**). In contrast, 3 out of 3 AAV9-eGFP treated mice showed a decrease in average telomere length and an increase in the percentage of short telomeres during the same time period (**Fig. 9****, panels A, B**). Together, these results indicate that forced TERT expression late in life by using a gene therapy approach results in rescue of short telomeres in several tissues and significant prevention of telomere shortening in peripheral blood lymphocytes from the same mice. This improved telomere maintenance maybe responsible for the systemic anti-ageing effects of telomerase and the increased survival observed in both groups of AAV9-mTERT injected mice.

### Example 6: Telomere expression is associated with increased β-catenin/cyclinD1 expression

Several cellular pathways have been demonstrated to modulate ageing (for comprehensive reviews see (Bishop, Lu *et al.*; Kenyon; Sahin and Depinho; Vaupel)), of which telomerase expression and telomere maintenance have wide experimental support from genetic studies in mice and humans (Blasco, Lee *et al.* 1997; Herrera, Samper *et al.* 1999; Blasco 2007; Tomas-Loba, Flores *et al.* 2008). Although TERT expression significantly prevents accumulation of short telomeres, it is also possible that at least part of the anti-ageing effects of TERT may be mediated by its recently proposed role as a cofactor on the promoter of Wnt targeted genes, which in turn is a key player in the self-renewing capabilities of adult stem cells (Choi, Southworth *et al.* 2008; Park, Venteicher *et al.* 2009). To assess this possibility, we determined the expression of the TERT/Wnt target genes β-catenin, Axin-2, CD44, as well as cyclin D1, a target of the Wnt pathway through β-catenin (Tetsu and McCormick 1999). We also included the expression of p16, a cell cycle inhibitor whose levels are dramatically increased associated with mouse ageing (Krishnamurthy, Ramsey *et al.* 2006; Molofsky, Slutsky *et al.* 2006).

Treatment with AAV9-mTERT lead to an increase in the active form of -catenin in all tissues tested, except for the liver (**Fig. 10****, panels A,B**). AAV9-mTERT treatment also lead to an increase in cyclin D1 positive cells compared to the control groups in all tissues with high AAV9 tropism (**Fig. 10****, panels C,D**), and this was also observed in the basal layer of the skin and neurogenic areas of the brain, such as the hippocampus (**Fig. 11**). The latter is of particular interest given the clear benefit of AAV9-mTERT treatment on cognitive tests (**see** **Fig. 4****, panels H-J**). Expression of other targets or effectors of the TERT/Wnt pathway, such as CD44 and axin2, however, were not consistent with TERT expression levels and where largely dependent on the tissue type, suggesting that TERT effects on some of the genes of the Wnt pathway may be regulated in a tissue specific manner **(****Fig.12****, panels A,B**).

Finally, consistent with better tissue fitness and delayed ageing of AAV9-mTERT treated mice, we observed decreased p16 levels in most organs studied (**Fig. 12****, panels C,D**) with the exception of the heart, which showed increased p16 levels as the result of TERT over-expression.

In summary, a number of cellular pathways involved in tissue renewing and stem cell fitness, such as Wnt and p16 pathways, are impacted after a pulse of mTERT, which in turn could contribute to increased healthspan and longevity of TERT treated mice.

### References

Armanios, M. Y., J. J. Chen, et al. (2007). "Telomerase mutations in families with idiopathic pulmonary fibrosis." N Engl J Med 356(13): 1317-26.
Artandi, S. E., S. Alson, et al. (2002). "Constitutive telomerase expression promotes mammary carcinomas in ageing mice." Proc-Natl Acad Sci USA 99(12): 8191-6.
Bailey, C. J. and P. R. Flatt (1982). "Hormonal control of glucose homeostasis during development and ageing in mice." Metabolism 31(3): 238-46.
Bevins, R. A. and J. Besheer (2006). "Object recognition in rats and mice: a one-trial non-matching-to-sample learning task to study 'recognition memory'." Nat Protoc 1(3): 1306-11.
Bishop, N. A., T. Lu, et al. "Neural mechanisms of ageing and cognitive decline." Nature 464(7288): 529-35.
Blasco, M. A. (2007). "Telomere length, stem cells and ageing." Nat Chem Biol 3(10): 640-9.
Blasco, M. A., H. W. Lee, et al. (1997). "Telomere shortening and tumour formation by mouse cells lacking telomerase RNA." Cell 91(1): 25-34.
Bluher, M., B. B. Kahn, et al. (2003). "Extended longevity in mice lacking the insulin receptor in adipose tissue." Science 299(5606): 572-4.
Bodnar, A. G., M. Ouellette, et al. (1998). "Extension of life-span by introduction of telomerase into normal human cells." Science 279(5349): 349-52.
Bonora, E., G. Targher, et al. (2000). "Homeostasis model assessment closely mirrors the glucose clamp technique in the assessment of insulin sensitivity: studies in subjects with various degrees of glucose tolerance and insulin sensitivity." Diabetes Care 23(1): 57-63.
Buning, H., L. Perabo, et al. (2008). "Recent developments in adeno-associated virus vector technology." J Gene Med 10(7): 717-33.
Calado, R. T. and N. S. Young (2009). "Telomere diseases." N Engl J Med 361(24): 2353-65.
Canela, A., J. Martin-Caballero, et al. (2004). "Constitutive expression oftert in thymocytes leads to increased incidence and dissemination of T-cell lymphoma in Lck-Tert mice." Mol Cell Biol 24(10): 4275-93.
Canela, A., E. Vera, et al. (2007). "High-throughput telomere length quantification by FISH and its application to human population studies." Proc Natl Acad Sci U S A 104(13): 5300-5.
Carter, B. J. (2005). "Adeno-associated virus vectors in clinical trials." Hum Gene Ther 16(5): 541-50.
Castelhano-Carlos, M. J., N. Sousa, et al. (2009). "Identification methods in newborn C57BL/6 mice: a developmental and behavioural evaluation." Lab Anim.
Conboy, I. M., M. J. Conboy, et al. (2005). "Rejuvenation of aged progenitor cells by exposure to a young systemic environment." Nature 433(7027): 760-4.
Counter, C. M., A. A. Avilion, et al. (1992). "Telomere shortening associated with chromosome instability is arrested in immortal cells which express telomerase activity." EMBO J 11(5): 1921-9.
Counter, C. M., W. C. Hahn, et al. (1998). "Dissociation among in vitro telomerase activity, telomere maintenance, and cellular immortalization." Proc Natl Acad Sci U S A 95(25): 14723-8.
Choi, J., L. K. Southworth, et al. (2008). "TERT promotes epithelial proliferation through transcriptional control of a Myc- and Wnt-related developmental program." PLoS Genet 4(1): e10.
de Boer, J., J. O. Andressoo, et al. (2002). "Premature ageing in mice deficient in DNA repair and transcription." Science 296(5571): 1276-9.
Fauce, S. R., B. D. Jamieson, et al. (2008). "Telomerase-based pharmacologic enhancement of antiviral function of human CD8+ T lymphocytes." J Immunol 181(10): 7400-6.
Ferguson, V. L., R. A. Ayers, et al. (2003). "Bone development and age-related bone loss in male C57BL/6J mice." Bone 33(3): 387-98.
Flores, I., A. Canela, et al. (2008). "The longest telomeres: a general signature of adult stem cell compartments." Genes Dev 22(5): 654-67.
Flores, I., M. L. Cayuela, et al. (2005). "Effects of telomerase and telomere length on epidermal stem cell behavior." Science 309(5738): 1253-6.
Flores, I., G. Evan, et al. (2006). "Genetic analysis of myc and telomerase interactions in vivo." Mol Cell Biol 26(16): 6130-8.
Garcia-Cao, I., M. Garcia-Cao, et al. (2002). ""Super p53" mice exhibit enhanced DNA damage response, are tumour resistant and age normally." EMBO J 21(22): 6225-35.
Garcia-Cao, I., M. Garcia-Cao, et al. (2006). "Increased p53 activity does not accelerate telomere-driven ageing." EMBO Rep 7(5): 546-52.
Gonzalez-Suarez, E., J. M. Flores, et al. (2002). "Cooperation between p53 mutation and high telomerase transgenic expression in spontaneous cancer development." Mol Cell Biol 22(20): 7291-301.
Gonzalez-Suarez, E., E. Samper, et al. (2001). "Increased epidermal tumours and increased skin wound healing in transgenic mice overexpressing the catalytic subunit of telomerase, mTERT, in basal keratinocytes." EMBO J 20(11): 2619-30.
Guarente, L. (2006). "Sirtuins as potential targets for metabolic syndrome." Nature 444(7121): 868-74.
Hammerman, M. R. (1987). "Insulin-like growth factors and ageing." Endocrinol Metab Clin North Am 16(4): 995-1011.
Harley, C. B., A. B. Futcher, et al. (1990). "Telomeres shorten during ageing of human fibroblasts." Nature 345(6274): 458-60.
Harrison, D. E., R. Strong, et al. (2009). "Rapamycin fed late in life extends lifespan in genetically heterogeneous mice." Nature 460(7253): 392-5.
Heikkinen, S., C. A. Argmann, et al. (2007). "Evaluation of glucose homeostasis." Curr Protoc Mol Biol Chapter 29: Unit 29B 3.
Hemann, M. T., M. A. Strong, et al. (2001). "The shortest telomere, not average telomere length, is critical for cell viability and chromosome stability." Cell 107(1): 67-77.
Herrera, E., E. Samper, et al. (1999). "Disease states associated with telomerase deficiency appear earlier in mice with short telomeres." EMBO J 18(11): 2950-60.
Inagaki, K., S. Fuess, et al. (2006). "Robust systemic transduction with AAV9 vectors in mice: efficient global cardiac gene transfer superior to that of AAV8." Mol Ther 14(1): 45-53.
Ingram, D. K. and M. A. Reynolds (1986). "Assessing the predictive validity of psychomotor tests as measures of biological age in mice." Exp Ageing Res 12(3): 155-62.
Jiang, H., E. Schiffer, et al. (2008). "Proteins induced by telomere dysfunction and DNA damage represent biomarkers of human ageing and disease." Proc Natl Acad Sci U S A 105(32): 11299-304.
Kenyon, C. J. "The genetics of ageing." Nature 464(7288): 504-12.
Krishnamurthy, J., M. R. Ramsey, et al. (2006). "p16INK4a induces an age-dependent decline in islet regenerative potential." Nature 443(7110): 453-7.
Lee, H. W., M. A. Blasco, et al. (1998). "Essential role of mouse telomerase in highly proliferative organs." Nature 392(6676): 569-74.
Lin J, Epel E, Cheon J, Kroenke C, Sinclair E, Bigos M, Wolkowitz O, Mellon S, Blackburn E (2010). "Analyses and comparisons of telomerase activity and telomere length in human T and B cells: insights for epidemiology of telomere maintenance"..J Immunol Methods. 31;352(1-2):71-80. Epub 2009 Oct 21
Matheu, A., A. Maraver, et al. (2007). "Delayed ageing through damage protection by the Arf/p53 pathway." Nature 448(7151): 375-9.
Matthews, D. R., J. P. Hosker, et al. (1985). "Homeostasis model assessment: insulin resistance and beta-cell function from fasting plasma glucose and insulin concentrations in man." Diabetologia 28(7): 412-9.
McKay, J. D., R. J. Hung, et al. (2008). "Lung cancer susceptibility locus at 5p15.33." Nat Genet 40(12): 1404-6.
Merten, O. W., C. Geny-Fiamma, et al. (2005). "Current issues in adeno-associated viral vector production." Gene Ther 12 Suppl 1: S51-61.
Millar, S. E. (2009). "Cell biology: The not-so-odd couple." Nature 460(7251): 44-5.
Mitchell, J. R., E. Wood, et al. (1999). "A telomerase component is defective in the human disease dyskeratosis congenita." Nature 402(6761): 551-5.
Molofsky, A. V., S. G. Slutsky, et al. (2006). "Increasing p16INK4a expression decreases forebrain progenitors and neurogenesis during ageing." Nature 443(7110): 448-52.
Moynihan, K. A., A. A. Grimm, et al. (2005). "Increased dosage of mammalian Sir2 in pancreatic beta cells enhances glucose-stimulated insulin secretion in mice." Cell Metab 2(2): 105-17.
Munoz, P., R. Blanco, et al. (2005). "XPF nuclease-dependent telomere loss and increased DNA damage in mice overexpressing TRF2 result in premature ageing and cancer." Nat Genet 37(10): 1063-71.
Ornish D, Lin J, Daubenmier J, Weidner G, Epel E, Kemp C, Magbanua MJ, Marlin R, Yglecias L, Carroll PR, Blackburn EH. (2008). "Increased telomerase activity and comprehensive lifestyle changes: a pilot study". Lancet Oncol. 9(11):1048-57. Epub 2008 Sep 15. Erratum in: Lancet Oncol. 2008 Dec;9(12):1124..
Parfitt, A. M. (1984). "Age-related structural changes in trabecular and cortical bone: cellular mechanisms and biomechanical consequences." Calcif Tissue Int 36 Suppl 1: S123-8.
Park, J. I., A. S. Venteicher, et al. (2009). "Telomerase modulates Wnt signalling by association with target gene chromatin." Nature 460(7251): 66-72.
Rafnar, T., P. Sulem, et al. (2009). "Sequence variants at the TERT-CLPTM1L locus associate with many cancer types." Nat Genet 41(2): 221-7.
Reya, T. and H. Clevers (2005). "Wnt signalling in stem cells and cancer." Nature 434(7035): 843-50.
Sahin, E. and R. A. Depinho "Linking functional decline of telomeres, mitochondria and stem cells during ageing." Nature 464(7288): 520-8.
Samper, E., J. M. Flores, et al. (2001). "Restoration of telomerase activity rescues chromosomal instability and premature ageing in Terc-/- mice with short telomeres." EMBO Rep 2(9): 800-7.
Sarin, K. Y., P. Cheung, et al. (2005). "Conditional telomerase induction causes proliferation of hair follicle stem cells." Nature 436(7053): 1048-52.
Schoeftner, S., R. Blanco, et al. (2009). "Telomere shortening relaxes X chromosome inactivation and forces global transcriptome alterations." Proc Natl Acad Sci U S A 106(46): 19393-8.
Shimokata, H., J. D. Tobin, et al. (1989). "Studies in the distribution of body fat: I. Effects of age, sex, and obesity." J Gerontol 44(2): M66-73.
Steen, B. (1988). "Body composition and ageing." Nutr Rev 46(2): 45-51.
Tafuro, S., E. Ayuso, et al. (2009). "Inducible adeno-associated virus vectors promote functional angiogenesis in adult organisms via regulated vascular endothelial growth factor expression." Cardiovasc Res 83(4): 663-71.
Tetsu, O. and F. McCormick (1999). "Beta-catenin regulates expression of cyclin D1 in colon carcinoma cells." Nature 398(6726): 422-6.
Tomas-Loba, A., I. Flores, et al. (2008). "Telomerase reverse transcriptase delays ageing in cancer-resistant mice." Cell 135(4): 609-22.
Tsakiri, K. D., J. T. Cronkhite, et al. (2007). "Adult-onset pulmonary fibrosis caused by mutations in telomerase." Proc Natl Acad Sci U S A 104(18): 7552-7.
Vaupel, J. W. "Biodemography of human ageing." Nature 464(7288): 536-42.
Vulliamy, T., A. Marrone, et al. (2001). "The RNA component of telomerase is mutated in autosomal dominant dyskeratosis congenita." Nature 413(6854): 432-5.
Yamaguchi, H., R. T. Calado, et al. (2005). "Mutations in TERT, the gene for telomerase reverse transcriptase, in aplastic anemia." N Engl J Med 352(14): 1413-24.
Zincarelli, C., S. Soltys, et al. (2008). "Analysis of AAV serotypes 1-9 mediated gene expression and tropism in mice after systemic injection." Mol Ther 16(6): 1073-80.

## Claims

1. A nucleic acid vector comprising a coding sequence for telomerase reverse transcriptase (TERT) for use in the treatment or prevention of an age-related disorder in an adult without the concomitant use of a cancer suppressor, wherein
the treatment does not significantly increase the incidence of cancer in the subject,
the nucleic acid vector is a non-integrative and post-mitotic tissue targeting vector,
the adult is a human
and
the age-related disorder is selected from the group of osteoporosis, glucose intolerance, insulin resistance, loss of memory, and loss of neuromuscular coordination, or combinations thereof.

2. A nucleic acid vector for use according to claim 1, wherein the treatment or preventive administration of the vector elicits an increase in lifespan.

3. A nucleic acid vector for use according to claim 1 or 2, wherein the vector is used in a gene therapy method.

4. A nucleic acid vector for use according to any one of claims 1 to 3, wherein TERT is encoded by the nucleic acid sequence as set forth in SEQ ID NO:1 or SEQ ID NO:3, or by an active fragment or functional equivalent thereof which encodes a polypeptide that has TERT activity.

5. A nucleic acid vector for use according to claim 4, wherein the functional equivalent is a nucleic acid having at least 80%, 90%, 95%, 99% or more sequence identity to the amino acid sequence as set forth in SEQ ID NO:1 or SEQ ID NO:3.

6. A nucleic acid vector for use according to any one of the preceding claims, wherein TERT comprises or consists of an amino acid sequence as set forth in SEQ ID NO:2 or SEQ ID NO:4, or is an active fragment or functional equivalent thereof that has TERT activity.

7. A nucleic acid vector for use according to claim 6, wherein the functional equivalent is a polypeptide having at least 80%, 90%, 95%, 99% or more sequence identity to the amino acid sequence as set forth in SEQ ID NO:2 or SEQ ID NO:4.

8. A nucleic acid vector for use according to any one of the preceding claims, wherein the nucleic acid sequence encoding TERT is operably linked to a regulatory sequence that drives the expression of the coding sequence.

9. A nucleic acid vector for use according to any one of the preceding claims, wherein the vector is an adeno-associated virus-based non-integrative vector.

10. A nucleic acid vector for use according to any one of the preceding claims, wherein the vector is an adeno-associated virus-based vector derived from a serotype 9 adeno-associated virus (AAV9).

11. A nucleic acid vector for use according to any one of the preceding claims, wherein the capsid of the adeno-associated virus-based vector is made of capsid proteins of the serotype 9 adeno-associated virus (AAV9), and the nucleic acid sequence contained in the capsid is flanked at both ends by internal terminal repeats corresponding to serotype 2 adeno-associated viruses.

12. A nucleic acid vector for use according to any one of the preceding claims, wherein the nucleic acid contained in the capsid comprises a fragment which encodes the amino acid sequence coding for TERT.

13. A nucleic acid vector for use according to any one of the preceding claims, wherein the regulatory sequence is a constitutive promoter.

14. A nucleic acid vector for use according to any one of the preceding claims, wherein the regulatory sequence is the cytomegalovirus (CMV) promoter.

15. A nucleic acid vector for use according to any of the preceding claims, wherein the adult is a human individual of at least 20 years of age or at least 25 years of age.

16. A nucleic acid vector for use according to claim 15, wherein the treatment or prevention of the aged-related disorder starts at 40, 45, 50, 60 years of age or older.

## Patentansprüche

1. Nucleinsäurevektor umfassend eine Codiersequenz für Telomerase Reverse Transkriptase (TERT) zur Verwendung bei der Behandlung oder Prävention einer altersbedingten Störung bei einem Erwachsenen ohne gleichzeitige Verwendung eines Krebsunterdrückers,
wobei
die Behandlung das Auftreten von Krebs bei dem Subjekt nicht signifikant erhöht,
der Nucleinsäurevektor ein nicht integrativer und postmitotischer Gewebetargetiervektor ist,
der Erwachsene ein Mensch ist
und
die altersbedingte Störung aus der Gruppe ausgewählt ist von Osteoporose, Glukoseintoleranz, Insulinresistenz, Gedächtnisverlust und Verlust neuromuskulärer Koordination und Kombinationen davon.

2. Nucleinsäurevektor zur Verwendung nach Anspruch 1, wobei die Behandlung oder präventive Verabreichung des Vektors eine Verlängerung der Lebensdauer hervorruft.

3. Nucleinsäurevektor zur Verwendung nach Anspruch 1 oder 2, wobei der Vektor bei einem Gentherapieverfahren verwendet wird.

4. Nucleinsäurevektor zur Verwendung nach einem der Ansprüche 1 bis 3, wobei TERT durch die wie in SEQ ID NO:1 oder SEQ ID NO:3 aufgeführte Nucleinsäuresequenz, oder durch ein aktives Fragment oder ein funktionelles Äquivalent davon, codiert wird, welches Äquivalent ein Polypeptid mit TERT Aktivität codiert.

5. Nucleinsäurevektor zur Verwendung nach Anspruch 4, wobei das funktionelle Äquivalent eine Nucleinsäure ist, die mindestens 80%, 90%, 95%, 99% oder mehr Sequenzidentität mit der wie in SEQ ID NO:1 oder SEQ ID NO:3 aufgeführten Aminosäuresequenz aufweist.

6. Nucleinsäurevektor zur Verwendung nach einem der vorhergehenden Ansprüche, wobei TERT eine wie in SEQ ID NO:2 oder SEQ ID NO:4 aufgeführte Aminosäuresequenz, oder ein aktives Fragment oder ein funktionelles, TERT-Aktivität aufweisendes Äquivalent davon, umfasst oder daraus besteht.

7. Nucleinsäurevektor zur Verwendung nach Anspruch 6, wobei das funktionelle Äquivalent ein Polypeptid ist, das mindestens 80%, 90%, 95%, 99% oder mehr Sequenzidentität mit der wie in SEQ ID NO:2 oder SEQ ID NO:4 aufgeführten Aminosäuresequenz aufweist.

8. Nucleinsäurevektor zur Verwendung nach einem der vorhergehenden Ansprüche, wobei die Nucleinsäuresequenz, die für TERT codiert, funktionsfähig mit einer regulatorischen Sequenz verknüpft ist, die die Expression der codierenden Sequenz steuert.

9. Nucleinsäurevektor zur Verwendung nach einem der vorhergehenden Ansprüche, wobei der Vektor ein auf adenoassoziierten Viren-basierender, nicht integrativer Vektor ist.

10. Nucleinsäurevektor zur Verwendung nach einem der vorhergehenden Ansprüche, wobei der Vektor ein auf adenoassoziierten Viren-basierender Vektor ist, der von einem adenoassoziierten Virus vom Serotyp 9 (AAV9) abgeleitet ist.

11. Nucleinsäurevektor zur Verwendung nach einem der vorhergehenden Ansprüche, wobei das Capsid des auf adenoassoziierten Viren-basiereden Vektors aus Capsidproteinen des adenoassoziierten Virus vom Serotyp 9 (AAV9) hergestellt ist und die Nucleinsäuresequenz, die in dem Capsid enthalten ist, an beiden Enden durch interne terminale Wiederholungen flankiert ist, die dem adenoassoziierten Virus vom Serotyp 2 entsprechen.

12. Nucleinsäurevektor zur Verwendung nach einem der vorhergehenden Ansprüche, wobei die Nucleinsäure, die in dem Capsid enthalten ist, ein Fragment umfasst, das die TERT codierende Aminosäuresequenz codiert.

13. Nucleinsäurevektor zur Verwendung nach einem der vorhergehenden Ansprüche, wobei die regulatorische Sequenz ein konstitutiver Promotor ist.

14. Nucleinsäurevektor zur Verwendung nach einem der vorhergehenden Ansprüche, wobei die regulatorische Sequenz der Cytomegalovirus-(CMV-) Promotor ist.

15. Nucleinsäurevektor zur Verwendung nach einem der vorhergehenden Ansprüche, wobei der Erwachsene ein menschliches Individuum im Alter von mindestens 20 Jahren oder im Alter von mindestens 25 Jahren ist.

16. Nucleinsäurevektor zur Verwendung nach Anspruch 15, wobei die Behandlung oder Prävention des altersbedingten Störung im Alter von 40, 45, 50, 60 Jahren oder darüber beginnt.

## Revendications

1. Vecteur d'acide nucléique comprenant une séquence de codage pour la transcriptase inverse de la télomérase (TERT) pour utilisation dans le traitement ou la prévention d'un trouble en rapport avec l'âge chez un adulte sans l'utilisation concomitante d'un suppresseur de cancer,
dans lequel
le traitement n'augmente pas significativement l'incidence du cancer chez le sujet,
le vecteur d'acide nucléique est un vecteur non intégratif et post-mitotique ciblant les tissus, l'adulte est un être humain et
le trouble en rapport avec l'âge est choisi dans le groupe de l'ostéoporose, de l'intolérance au glucose, de la résistance à l'insuline, de la perte de mémoire et de la perte de coordination neuromusculaire ou d'une de leurs combinaisons.

2. Vecteur d'acide nucléique pour utilisation selon la revendication 1, dans lequel le traitement ou l'administration préventive du vecteur entraîne une augmentation de la durée de vie.

3. Vecteur d'acide nucléique pour utilisation selon la revendication 1 ou la revendication 2, dans lequel le vecteur est utilisé dans un procédé de thérapie génique.

4. Vecteur d'acide nucléique pour utilisation selon l'une quelconque des revendications 1 à 3, dans lequel la TERT est codée par la séquence d'acide nucléique tel que mentionnée dans la SEQ ID N0:1 ou la SEQ ID NO:3 ou par un fragment actif ou un équivalent fonctionnel de celle-ci qui code pour un polypeptide qui a une activité TERT.

5. Vecteur d'acide nucléique pour utilisation selon la revendication 4, dans lequel l'équivalent fonctionnel est un acide nucléique ayant au moins 80 %, 90 %, 95 %, 99 % ou plus d'identité avec la séquence d'acides aminés telle que mentionnée dans la SEQ ID NO:1 ou la SEQ ID NO:3.

6. Vecteur d'acide nucléique pour utilisation selon l'une quelconque des revendications précédentes, dans lequel la TERT comprend ou consiste en une séquence d'acides aminés telle que mentionnée dans la SEQ ID NO:2 ou la SEQ ID NO:4, ou est un fragment actif ou un équivalent fonctionnel de celle-ci qui a une activité TERT.

7. Vecteur d'acide nucléique pour utilisation selon la revendication 6, dans lequel l'équivalent fonctionnel est un polypeptide ayant au moins 80 %, 90 %, 95 %, 99 % ou plus d'identité avec la séquence d'acides aminés telle que mentionnée dans la SEQ ID NO:2 ou la SEQ ID NO:4.

8. Vecteur d'acide nucléique pour utilisation selon l'une quelconque des revendications précédentes, dans lequel la séquence d'acide nucléique codant pour la TERT est liée opérationnellement à une séquence régulatrice qui entraîne l'expression de la séquence de codage.

9. Vecteur d'acide nucléique pour utilisation selon l'une quelconque des revendications précédentes, dans lequel le vecteur est un vecteur non intégratif à base de virus adéno-associé.

10. Vecteur d'acide nucléique pour utilisation selon l'une quelconque des revendications précédentes, dans lequel le vecteur est un vecteur à base de virus adéno-associé dérivé d'un virus adéno-associé de sérotype 9 (AAV9).

11. Vecteur d'acide nucléique pour utilisation selon l'une quelconque des revendications précédentes, dans lequel la capside du vecteur à base de virus adéno-associé est constituée de protéines de capside du virus adéno-associé de sérotype 9 (AAV9) et la séquence d'acide nucléique contenue dans la capside est flanquée aux deux extrémités par des répétitions terminales internes correspondant à des virus adéno-associés de sérotype 2.

12. Vecteur d'acide nucléique pour utilisation selon l'une quelconque des revendications précédentes, dans lequel l'acide nucléique contenu dans la capside comprend un fragment qui code pour la séquence d'acides aminés codant pour la TERT.

13. Vecteur d'acide nucléique pour utilisation selon l'une quelconque des revendications précédentes, dans lequel la séquence régulatrice est un promoteur constitutif.

14. Vecteur d'acide nucléique pour utilisation selon l'une quelconque des revendications précédentes, dans lequel la séquence régulatrice est le promoteur de cytomégalovirus (CMV).

15. Vecteur d'acide nucléique pour utilisation selon l'une quelconque des revendications précédentes, dans lequel l'adulte est un individu humain âgé d'au moins 20 ans ou d'au moins 25 ans.

16. Vecteur d'acide nucléique pour utilisation selon la revendication 15, dans lequel le traitement ou la prévention du trouble en rapport avec l'âge démarre à 40, 45, 50, 60 ans ou plus.
